(19) Europäisches Patentamt / European Patent Office / Office européen des brevets

(11) EP 1 678 299 B1

(12) # EUROPÄISCHE PATENTSCHRIFT

(45) Veröffentlichungstag und Bekanntmachung des Hinweises auf die Patenterteilung:
**02.07.2014 Patentblatt 2014/27**

(21) Anmeldenummer: **04802647.0**

(22) Anmeldetag: **22.10.2004**

(51) Int Cl.:
***C12N 15/10*** *(2006.01)*

(86) Internationale Anmeldenummer:
**PCT/DE2004/002386**

(87) Internationale Veröffentlichungsnummer:
**WO 2005/040376 (06.05.2005 Gazette 2005/18)**

(54) **VERFAHREN ZUR SELEKTION VON BIOMOLEKÜLEN AUS VARIANTEN-BIBLIOTHEKEN VON BIOMOLEKÜLEN**

METHOD FROM THE SELECTION OF BIOMOLECULES FROM BIOMOLECULE VARIANT LIBRARIES

PROCEDE POUR SELECTIONNER DES BIOMOLECULES DANS DES BANQUES DE VARIANTS DE BIOMOLECULES

(84) Benannte Vertragsstaaten:
**AT BE BG CH CY CZ DE DK EE ES FI FR GB GR HU IE IT LI LU MC NL PL PT RO SE SI SK TR**

(30) Priorität: **23.10.2003 DE 10350474**

(43) Veröffentlichungstag der Anmeldung:
**12.07.2006 Patentblatt 2006/28**

(73) Patentinhaber: **c-LEcta GmbH**
**04103 Leipzig (DE)**

(72) Erfinder:
- **GREINER-STÖFFELE, Thomas**
  **04317 Leipzig (DE)**
- **STRUHALLA, Marc**
  **04229 Leipzig (DE)**

(74) Vertreter: **Bülle, Jan et al**
**Kutzenberger Wolff & Partner**
**Theodor-Heuss-Ring 23**
**50668 Köln (DE)**

(56) Entgegenhaltungen:
**WO-A-01/12791**

- **MARC STRUHALLA: "Veränderung der Substratspezifität von Ribonuklease T1 und Einsatz des Enzyms in Immuntoxinen" 2003, UNIVERSITÄT HAMBURG , HAMBURG , XP002325208 das ganze Dokument**
- **HUBNER BERND ET AL: "Modification of ribonuclease T1 specificity by random mutagenesis of the substrate binding segment" BIOCHEMISTRY, Bd. 38, Nr. 4, 26. Januar 1999 (1999-01-26), Seiten 1371-1376, XP002325205 ISSN: 0006-2960**
- **KORN K ET AL: "Ribonuclease assays utilizing toluidine blue indicator plates, methylene blue, or fluorescence correlation spectroscopy." METHODS IN ENZYMOLOGY. 2001, Bd. 341, 2001, Seiten 142-153, XP009046414 ISSN: 0076-6879**
- **KORN KERSTIN ET AL: "Analysis of the RNase T1 mediated cleavage of an immobilized gapped heteroduplex via fluorescence correlation spectroscopy" BIOLOGICAL CHEMISTRY, Bd. 381, Nr. 3, März 2000 (2000-03), Seiten 259-263, XP002325207 ISSN: 1431-6730**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 678 299 B1

SEQUENZPROTOKOLL - SEQUENCE LISTING

<110> Universität Leipzig

<120> Verfahren zur Selektion von Biomolekülen aus Varianten-Bibliotheken von Biomolekülen

<130> 401P03DPCT

<150> DE10350474.5

<151> 2003-10-23
<160> 11
<170> PatentIn version 3.3
<210> 1
<211> 28
<212> DNA
<213> artificial
<400> 1
caattctgca gttgcgttca cgtcgttg 28
<210> 2
<211> 28
<212> DNA
<213> artificial
<400> 2
taaggctcat gaaaaacaca gctatcgc 28
<210> 3
<211> 378
<212> DNA
<213> Escherichia coli
<220>
<221> ompA-Signalpeptid
<222> (1)..(63)
<223>
<220>
<221> RNase T1 Wildtyp
<222> (64)..(378)
<223>
<400> 3

```
atgaaaaaca cagctatcgc gattgcagtg gcactggctg gtttcgctac cgtagcgcag      60
gccgcatgcg actacacttg cggttctaac tgctactctt cttcagacgt ttctactgct     120
caggcggccg gatataaact tcacgaagac ggtgaaactg ttggatccaa ttcttaccca     180
cacaagtaca acaactacga aggttttgat ttctctgtga gctctcccta ctacgaatgg     240
cctatcctct cgagcggtga tgtttactct ggtgggtccc cgggtgctga ccgtgtcgtc     300
ttcaacgaaa acaaccaact agctggtgtt atcactcaca ctggtgcttc tggtaacaac     360
ttcgttgaat gtacataa                                                   378
```

<210> 4
<211> 378
<212> DNA
<213> Escherichia coli
<220>
<221> ompA-Signalpeptid
<222> (1)..(63)
<223>
<220>
<221> RNaseT1-His92Ala
<222> (64)..(378)
<223>
<400> 4

```
atgaaaaaca cagctatcgc gattgcagtg gcactggctg gtttcgctac cgtagcgcag      60
gccgcatgcg actacacttg cggttctaac tgctactctt cttcagacgt ttctactgct     120
caggcggccg gatataaact tcacgaagac ggtgaaactg ttggatccaa ttcttaccca     180
cacaagtaca acaactacga aggttttgat ttctctgtga gctctcccta ctacgaatgg     240
cctatcctct cgagcggtga tgtttactct ggtgggtccc cgggtgctga ccgtgtcgtc     300
ttcaacgaaa acaaccaact agctggtgtt atcactgcca ctggtgcttc tggtaacaac     360
ttcgttgaat gtacataa                                                   378
```

<210> 5
<211> 7336
<212> DNA
<213> Plasmid pA2T1
<220>
<221> lac Promotor
<222> (1)..(371)<223>
<220>
<221> ompA-Signalpeptid
<222> (393)..(455)
<223>
<220>
<221> RNaseT1-Wildtyp
<222> (456).. (770)
<223>
<220>
<221> lacI-Gen
<222> (1664)..(2887)
<223>
<220>
<221> ORI
<222> (4924)..(5115)
<223>
<220>
<221> Beta-Lactamase (Amp)
<222> (7165..(6302))
<223>
<400> 5

```
taggcgtatc acgaggccct ttggataacc agaagcaata aaaaatcaaa tcggatttca      60
ctatataatc tcactttatc taagatgaat ccgatggaag catcctgttt tctctcaatt     120
tttttatcta aaacccagcg ttcgatgctt ctttgagcga acgatcaaaa ataagtgcct     180
```

tcccatcaaa aaaatattct caacataaaa aactttgtgt aatacttgta acgctacatg 240

gagattaact caatctagct agagaggctt tacactttat gcttccggct cgtataatgt 300

gtggaattgt gagcggataa caatttcaca caggaaacag ctatgaccat gattacggat 360

tcactggaac tctagataac gaggcgcaaa aaatgaaaaa cacagctatc gcgattgcag 420

tggcactggc tggtttcgct accgtagcgc aggccgcatg cgactacact tgtggttcca 480

actgctactc ttcttcagac gtttctactg ctcaagcggc cggatataaa cttcacgaag 540

acggtgaaac tgttggatcc aattcttacc cacacaaata caacaactac gaaggttttg 600

atttctctgt gagctctccc tactacgaat ggcctatcct ctcgagcggt gatgtttact 660

ctggtgggtc cccgggtgct gaccgtgtcg tcttcaacga aaacaaccaa ctagctggtg 720

ttatcactca cactggtgct tctggtaaca acttcgttga atgtacataa gcttggatcg 780

atccgggctg agcaacgacg tgaacgcaat gcgttccgac gttcaggctg ctaaagatga 840

cgcagctcgt gctaaccagc gtctggacaa catggctact aaataccgca agtaatagta 900

cctgtgaagt gaaaaatggc gcacattgtg cgacattttt tttgtctgcc gtttaccgct 960

actgcgtcac gcgtaacata ttcccttgct ctggttcacc attctgcgct gactctactg 1020

aaggcgcatt gctggctgcg ggagttgctc cactgctcac cgaaaccgga taccctgccc 1080

gacgatacaa cgctttatcg actaacttct gatctacagc cttattgtct ttaaattgcg 1140

taaagcctgc tggcagtgtg tatggcattg tctgaacgtt ctgctgttct cctgccgata 1200

gtggtcgatg tacttcaaca taacgcatcc cgttaggctc cacggaatat ttcaccggtt 1260

cgttgatcac tttcaccggc gttcccgtcc gcacgctgga gaacaaggct ttaatatccg 1320

gtgcattcat gcgaatacac cctgaactga cgcgcaaacc gacgctgtcc ggcgcactgg 1380

taccatgaat gaggtattcg ccattaccat gcgcgaggcg cagtgcgtaa cgtcctagcg 1440

ggttatttgg tccggcagga acgactggcg gtaatttaat gccacgctcc agcgaacgct 1500

gacgaatgcc tgccgtaggc gtccaggttg ggttagggat tttctgccca acacgcgttt 1560

ccatcaccgg cgtttccagc ccctgcaatc caatacctat tggataaacc tgcacaatat 1620

tttctcccgg cggataataa taaaggcgca gctctgcaag gttgacacca tcgaatggcg 1680

caaaaccttt cgcggtatgg catgatagcg cccggaagag agtcaattca gggtggtgaa 1740

tgtgaaacca gtaacgttat acgatgtcgc agagtatgcc ggtgtctctt atcagaccgt 1800

ttcccgcgtg gtgaaccagg ccagccacgt ttctgcgaaa acgcgggaaa aagtggaagc 1860

ggcgatggcg gagctgaatt acattcccaa ccgcgtggca caacaactgg cgggcaaaca 1920

gtcgttgctg attggcgttg ccacctccag tctggccctg cacgcgccgt cgcaaattgt 1980

cgcggcgatt aaatctcgcg ccgatcaact gggtgccagc gtggtggtgt cgatggtaga 2040
acgaagcggc gtcgaagcct gtaaagcggc ggtgcacaat cttctcgcgc aacgcgtcag 2100
tgggctgatc attaactatc cgctggatga ccaggatgcc attgctgtgg aagctgcctg 2160
cactaatgtt ccggcgttat ttcttgatgt ctctgaccag acacccatca acagtattat 2220
tttctcccat gaagacggta cgcgactggg cgtggagcat ctggtcgcat tgggtcacca 2280
gcaaatcgcg ctgttagcgg gcccattaag ttctgtctcg gcgcgtctgc gtctggctgg 2340
ctggcataaa tatctcactc gcaatcaaat tcagccgata gcggaacggg aaggcgactg 2400
gagtgccatg tccggttttc aacaaaccat gcaaatgctg aatgagggca tcgttcccac 2460
tgcgatgctg gttgccaacg atcagatggc gctgggcgca atgcgcgcca ttaccgagtc 2520
cgggctgcgc gttggtgcgg atatctcggt agtgggatac gacgataccg aagacagctc 2580
atgttatatc ccgccgtcaa ccaccatcaa acaggatttt cgcctgctgg ggcaaaccag 2640
cgtggaccgc ttgctgcaac tctctcaggg ccaggcggtg aagggcaatc agctgttgcc 2700
cgtctcactg gtgaaaagaa aaaccaccct ggcgcccaat acgcaaaccg cctctccccg 2760
cgcgttggcc gattcattaa tgcagctggc acgacaggtt tcccgactgg aaagcgggca 2820
gtgagcgcaa cgcaattaat gtgagttagc tcactcatta ggcaccccag gctttacact 2880
ttatgctaac gataatcccc tgacgcggtg catcaggtaa taacagttgt gaaggaatag 2940
ttatcgtcgt accaggtttt ggcaccgggg cgatagtgtt attggcttca aggatcaaca 3000
ttgccgcagt atcaaaacgt cgggcaatag cctgaaggtt tttatcccct tcttgcaccg 3060
tatacgtttg attttgccca accagtcggc ttccggttgg tggtagcgga taatcaaccg 3120
cccaggcagc ctggatggcg ctaaaagcgc cgataagcgt gagtgtaagc aaagacgcgc 3180
gtttcattgt aaacctcctg tatttgccgg agactcacgc tgaaacgtcg gatggcgctt 3240
atgttcacct gaaaccaaaa cactcctgtg caggtcagtg taaacattga ccatccggca 3300
atgtgagcca accggatgaa agctgtcctt ttagtttagc taagtgcagc ggctttggcg 3360
cgaattgcgc gaatcatcgc ttccagacct tgtgaacgag atggggtgag atgttgggtg 3420
agcgccattt tttcaaacca cggacgcaca tcgaaattga caatatcctg cggcgtcatc 3480
tgatcgtaga gaataaagac gaccgcaata agccctttca caatcgccgc atcgctgtcg 3540
ccctgtaatt caataattcc ctgggcattc tggcgcatga caatccacac ctgactctga 3600
cagccctgaa tgctattttg tggacttctg tcttcgtcgc gtaattctgg cagacgctgg 3660
gggaccgatg cccttgagag ccttcaaccc agtcagctcc ttccggtggg cgcggggcat 3720
gactatcgtc gccgcactta tgactgtctt ctttatcatg caactcgtag gacaggtgcc 3780
ggcagcgctc tgggtcattt tcggcgagga ccgctttcgc tggagcgcga cgatgatcgg 3840

cctgtcgctt gcggtattcg gaatcttgca cgccctcgct caagccttcg tcactggtcc 3900

cgccaccaaa cgtttcggcg agaagcaggc cattatcgcc ggcatggcgg ccgacgcgct 3960

gggctacgtc ttgctggcgt tcgcgacgcg aggctggatg gccttcccca ttatgattct 4020

tctcgcttcc ggcggcatcg ggatgcccgc gttgcaggcc atgctgtcca ggcaggtaga 4080

tgacgaccat cagggacagc ttcaaggatc gctcgcggct cttaccagcc taacttcgat 4140

cactggaccg ctgatcgtca cggcgattta tgccgcctcg gcgagcacat ggaacgggtt 4200

ggcatggatt gtaggcgccg ccctatacct tgtctgcctc cccgcgttgc gtcgcggtgc 4260

atggagccgg gccacctcga cctgaatgga agccggcggc acctcgctaa cggattcacc 4320

actccaagaa ttggagccaa tcaattcttg cggagaactg tgaatgcgca aaccaaccct 4380

tggcagaaca tatccatcgc gtccgccatc tccagcagcc gcacgcggcg catctcgggc 4440

agcgttgggt cctggccacg ggtgcgcatg atcgtgctcc tgtcgttgag gacccggcta 4500

ggctggcggg gttgccttac tggttagcag aatgaatcac cgatacgcga gcgaacgtga 4560

agcgactgct gctgcaaaac gtctgcgacc tgagcaacaa catgaatggt cttcggtttc 4620

cgtgtttcgt aaagtctgga aacgcggaag tcagcgccct gcaccattat gttccggatc 4680

tgcatcgcag gatgctgctg gctaccctgt ggaacaccta catctgtatt aacgaagcgc 4740

tggcattgac cctgagtgat ttttctctgg tcccgccgca tccataccgc cagttgttta 4800

ccctcacaac gttccagtaa ccgggcatgt tcatcatcag taacccgtat cgtgagcatc 4860

ctctctcgtt tcatcggtat cattaccccc atgaacagaa atccccctta cacggaggca 4920

tcagtgacca aacaggaaaa aaccgccctt aacatggccc gctttatcag aagccagaca 4980

ttaacgcttc tggagaaact caacgagctg gacgcggatg aacaggcaga catctgtgaa 5040

tcgcttcacg accacgctga tgagctttac cgcagctgcc tcgcgcgttt cggtgatgac 5100

ggtgaaaacc tctgacacat gcagctcccg gagacggtca cagcttgtct gtaagcggat 5160

gccgggagca gacaagcccg tcagggcgcg tcagcgggtg ttggcgggtg tcggggcgca 5220

gccatgaccc agtcacgtag cgatagcgga gtgtatactg gcttaactat gcggcatcag 5280

agcagattgt actgagagtg caccatatgc ggtgtgaaat accgcacaga tgcgtaagga 5340

gaaaataccg catcaggcgc tcttccgctt cctcgctcac tgactcgctg cgctcggtcg 5400

ttcggctgcg gcgagcggta tcagctcact caaaggcggt aatacggtta tccacagaat 5460

caggggataa cgcaggaaag aacatgtgag caaaaggcca gcaaaaggcc aggaaccgta 5520

aaaaggccgc gttgctggcg tttttccata ggctccgccc ccctgacgag catcacaaaa 5580

atcgacgctc aagtcagagg tggcgaaacc cgacaggact ataaagatac caggcgtttc 5640

cccctggaag ctccctcgtg cgctctcctg ttccgaccct gccgcttacc ggatacctgt 5700
ccgcctttct cccttcggga agcgtggcgc tttctcatag ctcacgctgt aggtatctca 5760
gttcggtgta ggtcgttcgc tccaagctgg gctgtgtgca cgaacccccc gttcagcccg 5820
accgctgcgc cttatccggt aactatcgtc ttgagtccaa cccggtaaga cacgacttat 5880
cgccactggc agcagccact ggtaacagga ttagcagagc gaggtatgta ggcggtgcta 5940
cagagttctt gaagtggtgg cctaactacg gctacactag aaggacagta tttggtatct 6000
gcgctctgct gaagccagtt accttcggaa aaagagttgg tagctcttga tccggcaaac 6060
aaaccaccgc tggtagcggt ggtttttttg tttgcaagca gcagattacg cgcagaaaaa 6120
aaggatctca agaagatcct ttgatctttt ctacggggtc tgacgctcag tggaacgaaa 6180
actcacgtta agggattttg gtcatgagat tatcaaaaag gatcttcacc tagatccttt 6240
taaattaaaa atgaagtttt aaatcaatct aaagtatata tgagtaaact tggtctgaca 6300
gttaccaatg cttaatcagt gaggcaccta tctcagcgat ctgtctattt cgttcatcca 6360
tagttgcctg actccccgtc gtgtagataa ctacgatacg ggagggctta ccatctggcc 6420
ccagtgctgc aatgataccg cgagacccac gctcaccggc tccagattta tcagcaataa 6480
accagccagc cggaagggcc gagcgcagaa gtggtcctgc aactttatcc gcctccatcc 6540
agtctattaa ttgttgccgg gaagctagag taagtagttc gccagttaat agtttgcgca 6600
acgttgttgc cattgctgca ggcatcgtgg tgtcacgctc gtcgtttggt atggcttcat 6660
tcagctccgg ttcccaacga tcaaggcgag ttacatgatc ccccatgttg tgcaaaaaag 6720
cggttagctc cttcggtcct ccgatcgttg tcagaagtaa gttggccgca gtgttatcac 6780
tcatggttat ggcagcactg cataattctc ttactgtcat gccatccgta agatgctttt 6840
ctgtgactgg tgagtactca accaagtcat tctgagaata gtgtatgcgg cgaccgagtt 6900
gctcttgccc ggcgtcaaca cgggataata ccgcgccaca tagcagaact ttaaaagtgc 6960
tcatcattgg aaaacgttct tcggggcgaa aactctcaag gatcttaccg ctgttgagat 7020
ccagttcgat gtaacccact cgtgcaccca actgatcttc agcatctttt actttcacca 7080
gcgtttctgg gtgagcaaaa acaggaaggc aaaatgccgc aaaaaaggga ataagggcga 7140
cacggaaatg ttgaatactc atactcttcc tttttcaata ttattgaagc atttatcagg 7200
gttattgtct catgagcgga tacatatttg aatgtattta gaaaaataaa caaatagggg 7260
ttccgcgcac atttccccga aaagtgccac ctgacgtcta agaaaccatt attatcatga 7320
cattaaccta taaaaa 7336

<210> 6
<211> 3653

<212> DNA
<213> Plasmid pETBlue-2
<220>
<221> T7-Promotor
<222> (1)..(17)
<223>
<220>
<221> lac Operator
<222> (22)..(42)
<223>
<220>
<221> f1 ORI
<222> (1096)..(1551)
<223>
<220>
<221> Beta-Lactamase (Amp)
<222> (2556)..(1669)
<223>
<220>
<221> pUC ORI
<222> (3206)..(3250)
<223>
<220>
<221> lac Operator
<222> (3606)..(3625)
<223>
<400> 6

```
taatacgact cactataggg gaattgtgag cggataacaa ttcccctcta gacttacaat    60
ttccattcgc cattcaggct gcgcaactgt tgggaagggc gatcggtacg ggcctcttcg   120
ctattacgcc agcttgcgaa cggtgggtgc gctgcaaggc gattaagttg ggtaacgcca   180
ggattctccc agtcacgacg ttgtaaaacg acggccagcg agagatcttg attggctagc   240
agaataattt tgtttaactt taagaaggag atataccatg gcgatatccc gggagctcgt   300
ggatccgaat tctgtacagg cgcgcctgca ggacgtcgac ggtaccatcg atacgcgttc   360
gaagcttgcg gccgcacagc tgtatacacg tgcaagccag ccagaactcg ctcctgaaga   420
cccagaggat ctcgagcacc accaccacca ccactaatgt taattaagtt gggcgttgta   480
atcatagtca taatcaatac tcctgactgc gttagcaatt taactgtgat aaactaccgc   540
attaaagcta ttcgatgata agctgtcaaa catgataatt cttgaagacg aaagggccta   600
ggctgataaa acagaatttg cctggcggca gtagcgcggt ggtcccacct gaccccatgc   660
cgaactcaga agtgaaacgc cgtagcgccg atggtagtgt ggggtctccc catgcgagag   720
tagggaactg ccaggcatca aataaaacga aaggctcagt cgaaagactg ggcctttcgt   780
tttatctgtt gtttgtcggt gaacgctctc ctgagtagga caaatccgcc gggagcggat   840
ttgaacgttg cgaagcaacg gcccggaggg tggcgggcag gacgcccgcc ataaactgcc   900
aggcatcaaa ttaagcagaa ggccatcctg acggatggcc tttttgcgtt tctacaaact   960
```

cttttgttta tttttctaaa tacattcaaa tatgtatccg ctgagcaata actagcataa 1020
ccccttgggg cctctaaacg ggtcttgagg ggttttttgc tgaaaggagg aactatatcc 1080
ggattggcga atgggacgcg ccctgtagcg gcgcattaag cgcggcgggt gtggtggtta 1140
cgcgcagcgt gaccgctaca cttgccagcg ccctagcgcc cgctcctttc gctttcttcc 1200
cttcctttct cgccacgttc gccggctttc cccgtcaagc tctaaatcgg gggctccctt 1260
tagggttccg atttagtgct ttacggcacc tcgaccccaa aaaacttgat tagggtgatg 1320
gttcacgtag tgggccatcg ccctgataga cggtttttcg ccctttgacg ttggagtcca 1380
cgttctttaa tagtggactc ttgttccaaa ctggaacaac actcaaccct atctcggtct 1440
attcttttga tttataaggg attttgccga tttcggccta ttggttaaaa aatgagctga 1500
tttaacaaaa atttaacgcg aattttaaca aaatattaac gtttacaatt tctggcggca 1560
cgatggcatg agattatcaa aaaggatctt cacctagatc cttttaaatt aaaaatgaag 1620
ttttaaatca atctaaagta tatatgagta aacttggtct gacagttacc aatgcttaat 1680
cagtgaggca cctatctcag cgatctgtct atttcgttca tccatagttg cctgactccc 1740
cgtcgtgtag ataactacga tacgggaggg cttaccatct ggccccagtg ctgcaatgat 1800
accgcgagac ccacgctcac cggctccaga tttatcagca ataaaccagc cagccggaag 1860
ggccgagcgc agaagtggtc ctgcaacttt atccgcctcc atccagtcta ttaattgttg 1920
ccgggaagct agagtaagta gttcgccagt taatagtttg cgcaacgttg ttgccattgc 1980
tacaggcatc gtggtgtcac gctcgtcgtt tggtatggct tcattcagct ccggttccca 2040
acgatcaagg cgagttacat gatcccccat gttgtgcaaa aaagcggtta gctccttcgg 2100
tcctccgatc gttgtcagaa gtaagttggc cgcagtgtta tcactcatgg ttatggcagc 2160
actgcataat tctcttactg tcatgccatc cgtaagatgc ttttctgtga ctggtgagta 2220
ctcaaccaag tcattctgag aatagtgtat gcggcgaccg agttgctctt gcccggcgtc 2280
aatacgggat aataccgcgc cacatagcag aactttaaaa gtgctcatca ttggaaaacg 2340
ttcttcgggg cgaaaactct caaggatctt accgctgttg agatccagtt cgatgtaacc 2400
cactcgtgca cccaactgat cttcagcatc ttttactttc accagcgttt ctgggtgagc 2460
aaaaacagga aggcaaaatg ccgcaaaaaa gggaataagg gcgacacgga aatgttgaat 2520
actcatactc ttcctttttc aatcatgacc aaaatccctt aacgtgagtt ttcgttccac 2580
tgagcgtcag accccgtaga aaagatcaaa ggatcttctt gagatccttt ttttctgcgc 2640
gtaatctgct gcttgcaaac aaaaaaacca ccgctaccag cggtggtttg tttgccggat 2700
caagagctac caactctttt tccgaaggta actggcttca gcagagcgca gataccaaat 2760
actgtccttc tagtgtagcc gtagttaggc caccacttca agaactctgt agcaccgcct 2820

acatacctcg ctctgctaat cctgttacca gtggctgctg ccagtggcga taagtcgtgt 2880
cttaccgggt tggactcaag acgatagtta ccggataagg cgcagcggtc gggctgaacg 2940
ggggggttcgt gcacacagcc cagcttggag cgaacgacct acaccgaact gagataccta 3000
cagcgtgagc tatgagaaag cgccacgctt cccgaaggga gaaaggcgga caggtatccg 3060
gtaagcggca gggtcggaac aggagagcgc acgagggagc ttccaggggg aaacgcctgg 3120
tatctttata gtcctgtcgg gtttcgccac ctctgacttg agcgtcgatt tttgtgatgc 3180
tcgtcagggg ggcggagcct atggaaaaac gccagcaacg cggccttttt acggttcctg 3240
gccttttgct ggccttttgc tcacatgttc tttcctgcgt tatcccctga ttctgtggat 3300
aaccgtatta ccgcctttga gtgagctgat accgctcgcc gcagccgaac gaccgagcgc 3360
agcgagtcag tgagcgagga agccggcgat aatggcctgc ttctcgccga aacgtttggt 3420
ggcgggacca gtgacgaagg cttgagcgag ggcgtgcaag attccgaata ccgcaagcga 3480
caggccgatc atcgtcgcgc tccagcgaaa gcggtcctcg ccgaaaatga cccagagcgc 3540
tgccggcacc tgtcctacga gttgcatgat aaagaagaca gtcataagtg cggcgacgac 3600
cggtgaattg tgagcgctca caattctcgt gacatcataa cgtcccgcga aat 3653

<210> 7
<211> 18
<212> DNA
<213> artificial
<400> 7
gtggctggct ggtatgga 18
<210> 8
<211> 18
<212> DNA
<213> artificial
<400> 8
tatctgtgct tcggtggc 18
<210> 9
<211> 98
<212> DNA
<213> artificial
<220>
<223> Primer Loop1_32
<220>
<223> compositions of n and b are further specified in the description
<400> 9

gtaggatcca attcttaccc acacnnbnnb nnbnnbnnbn nbnnbnnbnn bnnbnnbnnb 60
nnbnnbnnbn nbnnbgaatg gcctatcctc tcgagcgg 98

<210> 10
<211>
<212> DNA

<213> artificial
<220>
<223> substrate "Sub_G"
<220>
<223> the g at position 24 is a ribonucleotide
<400> 10
ccataccagc cagccacaag caagccaccg aagcacagat a 41
<210> 11
<211>
<212> DNA
<213> artificial
<220>
<223> substrate "Sub_A"
<220>
<223> the a at position 24 is a ribonucleotide
<400> 11
ccataccagc cagccacaag caaaccaccg aagcacagat a 41

## Beschreibung

Die Erfindung betrifft ein Verfahren zur Selektion von Biomolekülen aus Varianten-Bibliotheken von Biomolekülen, insbesondere von Enzymen oder anderen biokatalytisch aktiven Biomolekülen. Biomoleküle finden vielseitige Verwendung in technologischen oder medizinischen Anwendungen und Prozessen. Viele der dafür benötigten Eigenschaften der Biomoleküle sind in der Natur so nicht vorhanden oder noch nicht identifiziert worden. Die Generierung solcher neuen Eigenschaften aus vorhandenen Biomolekülen erfordert die Herstellung sehr großer Varianten-Bibliotheken mit zufällig veränderten Zusammensetzungen durch Einführung von Mutationen. Die Identifizierung der Varianten mit den gesuchten Eigenschaften erfordert geeignete Selektions- oder Durchmusterungs-Verfahren.

So wurde beispielsweise die Veränderung der Substratspezifität von Ribonuklease T1 durch zufällige Mutationen des Substrat Bindungssegments sowie die Veränderung der Substratspezifität von Ribonuklease T1 und der Einsatz des Enzyms in Immuntoxinen untersucht (Hubner, B. et al. Modification of Ribonuclease T1 specificity by random mutagenesis of the substrate binding segment. Biochemistry. 1999. 38. 1371-6. Marc, S. Veränderung der Substratspezifität von Ribonuklease T1 und der Einsatz des Enzyms in Immuntoxinen. 2003. Universität Hamburg).

Es wurden z.B. Ribonuklease Assays beschrieben, welche Toluidin-Blau Indikator Platten, Methylen Blau oder Fluoreszenz Korrelationsspektroskopie verwenden. Korrelationsspektroskopie wurde verwendet, um die durch Rnase T1 vermittelte Spaltung eines immobilisierten Heteroduplex zu analysieren (Korn, K. et al. Analysis of the Rnase T1 mediated cleavage of an immobilized gapped heteroduplex via fluorescence correlation spectroscopy. Biological Chemistry. 2000. 381. 259-63. Korn, K. et al. Ribonuclease assays utilizing toluidine blue indicator plates, methylene blue, or fluorescence correlation spectroscopy. Methods in Enzymology. 2001. 341. 142-53).

Die Verbesserung der Eigenschaften von Biomolekülen, im Speziellen von Proteinen, können z.B. die Regioselektivität, die enzymatische Aktivität, die Stereospezifität und der gleichen betreffen. So wurden beispielsweise durch "DNA shüffling" veränderte Dioxygenasen offenbart (WO 01/12791).

Das zufällige Einführen von Mutationen in das genetische Material ist auch die Triebfeder der natürlichen Evolution. Natürliche Systeme replizieren dabei mit Mutationsraten, die gerade knapp unter der so genannten Fehlerschwelle liegen. Die Fehlerschwelle ist dabei die maximale Mutationsrate, die gerade nicht zum Aussterben der Population führt. Bei Mutationsraten unterhalb der Fehlerschwelle werden genügend Variationen in einer Population angehäuft, um der Population eine schnell Anpassung an veränderte Bedingungen zu ermöglichen. Mutationsraten über der Fehlerschwelle führen hingegen nach einigen Generationen dazu, dass keine überlebensfähigen bzw. replizierbaren Individuen mehr vorhanden sind, und die Population zusammenbricht (Eigen, M., McCaskill, J., Schuster, P.: The molecular quasispecies. Adv. Chem. Phys. 1989. 75. 149-263).

Neue Biomoleküle können durch die Verknüpfung der neuen Eigenschaft an das Überleben oder einen hinreichend großen Wachstumsvorteil eines Organismus erzeugt werden. Hierbei wird die Varianten-Bibliothek in einen entsprechenden Organismus überführt und die Wachstumsbedingungen so gewählt, dass nur die Individuen überleben oder vergleichsweise schneller wachsen, welche eine Variante des Biomoleküls mit der gesuchten neuen Eigenschaft produzieren (Zaccolo M, Gherardi E. The effect of high-frequency random mutagenesis on in vitro protein evolution: a study on TEM-1 beta-lactamase. J. Mol. Biol. 1999. 285, 775-83. oder Samuelson JC, Xu SY. Directed evolution of restriction endonuclease Butyl to achieve increased substrate specificity. J. Mol. Biol. 2002. 319,673-83). Diese Anwendung ist nur auf die Selektion eines eng begrenzten Kreis von Biomolekülen anwendbar, die einem gewählten Organismus einen Vorteil verschaffen können. Biomoleküle, welche beliebige chemische Reaktionen katalysieren, sind so nicht selektierbar. Da der Organismus über den gesamten Selektionszeitraum am Leben bleiben muss, sind toxische oder anderweitig für ein Wachstum nachteilige Eigenschaften nicht selektierbar.

Eine weitere Methode zur Selektion neuer Biomoleküle ist die Verknüpfung des Biomoleküls mit der codierenden Nukleinsäure-Sequenz (Amstutz P, Forrer P, Zahnd C, Pluckthun A. In vitro display technologies: novel developments and applications. Curr. Opin.Biotechnol. 2001. 12. 400-5. Xia G, Chen L, Sera T, Fa M, Schultz PG, Romesberg FE. Directed evolution of novel polymerase activities: mutation of a DNA polymerase into an efficient RNA polymerase. Proc. Natl. Acad. Sci. USA. 2002. 99. 6597-602. Pschorr J. Genotyp und Phänotyp koppelnde Verbindung. DE0019646372C1). Eine Anwendung dieser Technologien mit lebenden Organismen wie Phagen oder Bakterien beschränkt das Spektrum wiederum auf nicht toxische oder nicht wachstumshemmende Biomoleküle. Ebenso dürfen Substrate und Produkte der gesuchten Reaktion auf den präsentierenden Organismus nicht schädigend einwirken. Zusätzlich lassen sich katalytische Aktivitäten nur selektieren, wenn sich Biomolekül und Substrat an dem gleichen Organismus präsentieren lassen. Da sich die Aktivität der katalytischen Biomoleküle nicht auf den sie präsentierenden Organismus begrenzen lässt und diese somit auch Reaktionen an anderen Individuen der Bibliothek stattfinden können, führt diese Methode häufig zur Falsch-Selektion von Biomolekülen.

Bei den Durchmusterungs-Verfahren (Screening-Verfahren) wird jede Variante einer Biomolekül-Bibliothek einzeln hinsichtlich der gesuchten Eigenschaft untersucht. (Joo H, Lin Z, Arnold FH. Laboratory evolution of peroxide-mediated cytochrome P450 hydroxylation. Nature. 1999. 399. 670-3. Korbel GA, Lalic G, Shair MD. Reaction microarrays: a method for rapidly determining the enantiomeric excess of thousands of samples. J. Am. Chem. Soc. 2001. 123.

361-2.) Selbst bei sehr kurzen Messzeiten (z. B. 100 ms pro Variante) erfordert diese Methode einer sehr hohen Zeitaufwand (z.B. 22 Tage) für die Untersuchung großer Bibliotheken (z.B. $10^7$). Das kontinuierliche Messen von Varianten in diesen Größenordnungen erfordert den Aufbau entsprechend komplexer Apparate. Außerdem muss für jede Variante der Bibliothek ein entsprechender Eigenschaftstest durchgeführt werden, was zu sehr hohen Kosten dieser Methoden führt.

Zum Screenen oder zur Veränderung von Enzymeigenschaften im Labor, dem sogenannten "Enzym Engineering" müssen nach dem Stand der Technik in einer Enzymbibliothek Genotyp (eine Nukleinsäure, die vervielfältig werden kann und eine Variante eines Gens umfasst) und Phänotyp (ein funktionales Merkmal, wie z. B. eine katalytische Eigenschaft) aneinander gekoppelt werden. Diese Kopplung wird z. B. durch Techniken, wie Phage-Display oder Ribosomen-Display erreicht oder dadurch, dass jeder Genotyp einzeln auf einen Phänotyp getestet wird.

Aufgabe der vorliegenden Erfindung ist es ein Verfahren zur Identifizierung von Biomolekülen in Varianten-Bibliotheken von Biomolekülen anzugeben.

Erfindungsgemäß wird die Aufgabe gelöst durch ein Verfahren zur Selektion von Biomolekülen aus Varianten-Bibliotheken von Biomolekülen umfassend die Schritte: .

b) Aufteilung einer Varianten-Bibliothek, bestehend aus einer Anzahl ($B_0$) von Varianten in einem Größenbereich von $B_0 = 10^3$ bis $B_0 = 10^{15}$ der für das Biomolekül codierenden Gensequenz in eine Anzahl von $10^1$ bis $10^4$ Kompartimenten (Wo), die mindestens um einen Faktor 10 kleiner ist als die Anzahl ($B_0$) der in der Varianten-Bibliothek enthaltenen Varianten, wobei jedes Kompartiment eine Teilbibliothek enthält, die $K_0 \approx B_0$ / Wo Varianten enthält;

c) Vervielfältigung der Teilbibliothek in den Kompartimenten bis zu einer Individuen-Anzahl $V_0(x)$ zum Zeitpunkt x pro Kompartiment, wobei die Individuen-Anzahl $V_0(x)$ dividiert durch die Klonanzahl pro Kompartiment $K_0$ den Vervielfältigungsfaktor $F_0(x)$ pro Klon ergibt;
Produktion von Biomolekülen in den Kompartimenten vor, während oder nach der Vervielfältigung der Genotypen;
Konservierung eines Anteils der Teilbibliothek auf Organismen-Ebene oder der Ebene reiner codierender Sequenzen zum Zeitpunkt x unter Beibehaltung der Kompartiment Zuordnung; und
Test der in den einzelnen Kompartimenten erhaltenen Biomoleküle auf eine bestimmte Eigenschaft (Phänotyp), wobei Genotyp und Phänotyp entkoppelt sind und aus dem beobachteten Phänotyp keine direkten Rückschlüsse auf den Genotyp möglich sind;

d) Auswahl mindestens eines Kompartiments, in dem Biomoleküle enthalten sind, welche die gewünschten Eigenschaften erfüllen;

e) Verdünnung der entsprechend konservierten Teilbibliothek anhand des Faktors $F_0(x)$, so dass in einem gegebenen Volumen jedes in dem Kompartiment enthaltene Klon statistisch bis zu einer Anzahl $X_0 < W_1$ vorkommt; und Aufteilung dieses Volumens ohne vorherige Vervielfältigung auf neue Kompartimente $W_1$, wobei die neue Klonanzahl pro Kompartiment $K_1 = X_0 * K_0/W_1$ beträgt; und

f) n-faches Wiederholen der Schritte c) bis e), bis in jedem Kompartiment nur noch maximal eine Variante ($K_n \leq 1$) der für das Biomolekül codierenden Gensequenz enthalten ist.

Dieses Verfahren ist insbesondere zur Generierung von Biomolekülen mit neuen katalytischen Aktivitäten geeignet, die entweder in der Natur gar nicht vorkommen oder zumindest von dem gewählten Ausgangs-Biomolekül so nicht katalysiert werden. Außerdem können mit diesem Verfahren vorhandene katalytische Aktivitäten an äußere Bedingungen, wie z.B. Temperatur oder Lösungsmittel, angepasst werden, unter denen bisher keine oder nur verschwindend geringe Aktivität vorhanden war.

Da eine Produktion der Biomoleküle in der vorliegenden Erfindung zu einem Absterben der Organismen führen kann oder durch zellfreie Systeme erfolgen kann, kann das Verfahren auf alle Arten von Biomoleküle angewandt werden und ist nicht auf nicht toxische oder nicht wachstumshemmende Aktivitäten begrenzt. Da bis zu einer Million oder mehr Varianten mit einem Test und gleichzeitig auf die entsprechende Eigenschaft hin untersucht werden, reduziert sich die für das Durchmustern der Bibliothek benötigten Zeit und die für die Eigenschaftstests benötigten Kosten um einen entsprechenden Faktor. Varianten, welche die gesuchten Eigenschaften aufweisen, können sicher und reproduzierbar aus den ursprünglichen Varianten-Gemischen isoliert werden.

Im Schritt a.) des Verfahrens wird eine Varianten-Bibliothek der für das Biomolekül codierenden Gensequenz mittels Standardmethoden der Molekularbiologie hergestellt. Unter Variantenbibliothek wird im Sinne der vorliegenden Erfindung verstanden: Mischung aus Proteinen oder Nukleinsäuren, welche sich in mindestens einer Position in ihrer Sequenz voneinander unterscheiden.

Die Variantenbibliothek besteht aus einer Anzahl von Varianten in einem Größenbereich von $B_0 = 10^3$ bis $B_0$

= $10^{15}$. So können zum Beispiel in einen Teilbereich des Biomoleküls zufällig gewählte Sequenzbausteine eingeführt werden, so dass im Fall einer Nukleinsäure bei 25 geänderten Positionen eine Bibliotheksgröße von $4^{25}$ = 1,1 x $10^{15}$ oder im Fall eines Proteins bei 7 geänderten Positionen eine Bibliotheksgröße von $20^7$ =1,3 x $10^9$ entstehen kann.

Besonders bevorzugt liegt der Größenbereich zwischen $B_0 = 10^5$ bis $B_0 = 10^9$.

Besonders bevorzugt besteht die Variantenbibliothek aus DNA-Plasmiden oder linearen Nukleinsäuremolekülen, welche die für das Biomolekül codierende Gensequenz enthalten.

Biomoleküle im Sinne des vorliegenden Erfindung sind Proteine, Nukleinsäuren oder andere aus organischen Bausteinen bestehende Biopolymere. Bevorzugt sind diese Biomoleküle, Enzyme oder Ribozyme oder andere Biomoleküle, die als Biokatalysatoren, die Umsetzung chemischer oder biochemischer Stoffe beschleunigen können.

Standardmethoden der Molekularbiologie mit denen eine solche Variantenbibliothek hergestellt werden kann, sind beispielsweise fehlerhafte Vervielfältigungsmethoden für Nukleinsäuren. Hierfür werden replizierende Enzyme, z.B. Polymerasen, welche die Neusynthese eines Biomoleküls mit Hilfe einer Matrize durchführen, angewandt. Die Einführung von Fehlern und somit die Erzeugung von unterschiedlichen Varianten erfolgt entweder durch die natürlich vorhandene Fehlerrate dieser replizierenden Enzyme oder kann durch Veränderung der Reaktionsbedingungen (z.B. Ungleichgewicht der Synthesebausteine, Zugabe von Baustein-Analoga, Veränderung der Puffer-Zusammensetzung) erhöht werden. Neben der Einführung von Fehlern kann eine Variantenbibliothek unter Ausnutzung der natürlich vorkommenden Diversität für ein bestimmtes Biomolekül oder eine Biomolekülklasse erzeugt werden.

Im Vergleich zu herkömmlichen Screeningverfahren erlaubt dass erfindungsgemäße Verfahren das Screening von sehr großen Bibliotheken. Das erfindungsgemäße Aufteilungsverfahren ermöglicht das gleichzeitige Testen von beliebig vielen Varianten.

Limitiert wird die Größe der Bibliothek nur durch die Sensitivität des Assays, mit dem die in den einzelnen Kompartimenten erhaltenen Biomoleküle auf eine bestimmte Eigenschaft, bevorzugt eine biokatalytische Aktivität, in Schritt c.) des Verfahrens getestet werden.

Vorzugsweise werden die Bibliotheken durch fehlerhafte PCR oder das Einfügen synthetisch randomisierter Sequenzabschnitte hergestellt (Cadwell RC, Joyce GF. Randomization of genes by PCR mutagenesis. PCR Methods Appl. 1992. 2. 28-33; (Wells JA, Vasser M, Powers DB. Cassette mutagenesis: an efficient method for generation of multiple mutations at defined sites. Gene. 1985. 34. 315-23).

Die Mutationsrate wird dabei bevorzugt deutlich über der Fehlerschwelle gewählt. Dadurch sind in der Ausgangsbibliothek bevorzugt mehr als 90 % , bevorzugt mehr als 99 % und besonders bevorzugt mehr als 99,9 % der erstellten Varianten nicht überlebensfähig sind.

Die Fehlerschwelle ist definiert als die maximale Mutationsrate, die bei evolutionären Methoden (zyklische Anwendung von Mutation und Selektion) gerade nicht zu einem Zerfließen der genetischen Information führt und somit die Überlebensfähigkeit einer Population erhält. Ein Zerfließen der genetischen Information wird definiert als ein Vorgang, bei dem durch wiederholtes Anlegen einer zu hohen Mutationsrate beim Replizieren einer Nukleinsäuresequenz sich so viele Mutationen anhäufen, dass die Nukleinsäure dann keine physiologisch sinnvolle Information mehr enthält.

Überlebensfähigkeit für ein Gen bzw. Genprodukt wird dabei so definiert, dass das Gen bzw. sein Genprodukt seine physiologische Aktivität wie zum Beispiel die Bildung eines Partners oder die katalytische Spaltung eines Substrates noch wahrnehmen kann.

Ein wichtiger Vorteil des erfindungsgemäßen Verfahren gegenüber etablierten Verfahren, die Mutagenese- und Selektionsschritte enthalten, besteht darin, dass im erfindungsgemäßen Verfahren von einer großen Bibliothek ausgegangen wird, die von vornherein die gewünschte Variante enthält. D. h. man erhält nach dem Screening nicht eine suboptimale Variante, die durch weitere Mutations- oder Rekombinationszyklen weiter verbessert werden muss.

Das erfindungsgemäße Verfahren zeichnet sich dadurch aus, dass zu Beginn einmalig im Schritt a.) eine Varianten-Bibliothek erstellt wird, welche im Anschluss auf Varianten mit der gewünschten Eigenschaft durchmustert wird. Ab dem Schritt b.) erfolgen keine weiteren Mutagenese oder Rekombinationschritte. D. h. zwischen oder während den einzelnen Vereinzelungsschritten (Schritte b. bis f.) werden die dabei isolierten Teilbibliotheken keiner weiteren Mutagenese oder Rekombination unterzogen. D. h. die am Ende des Verfahrens isolierten Varianten mit den gewünschten Eigenschaften sind bereits in der initialen (in Schritt a.) eingesetzten Bibliothek vorhanden.

Bevorzugt wird das erfindungsgemäße Verfahren so durchgeführt, dass in Schritt d.) in allen Durchgängen nur ein Kompartiment ausgewählt wird und zwar das in dem die gewünschte Eigenschaft (Phänotyp) am stärksten ausgeprägt ist, vorzugsweise das Kompartiment mit der stärksten biokatalytischen Aktivität. Dabei kann mit dem erfindungsgemäßen Verfahren die beste Variante isoliert werden, in der die gewünschte Eigenschaft (Phänotyp) am stärksten ausgeprägt ist, ohne dass eine Auswahl von suboptimalen Varianten oder von Gruppen von Varianten zwingend erforderlich ist.

Bei dem Erstellen der Variantenbibliothek wird vorzugsweise von einer bereits bekannten Nukleinsäure- oder Proteinsequenz ausgegangen, nachfolgend Ausgangssequenz genannt. Basierend auf dieser Ausgangssequenz wird mit den oben genannten Methoden (z. B. fehlerhafter PCR oder durch das Einfügen synthetisch randomisierter Sequenzabschnitte) die Variantenbibliothek hergestellt

Das erfindungsgemäße Verfahren zeichnet sich weiterhin dadurch aus, dass die Ausgangssequenz nicht mehr in der Variantenbibliothek enthalten sein muss.

Die Ausgangsequenz codiert häufig für einen Phänotyp der der gewünschten Eigenschaft zu einem gewissen Grade ähnlich ist. So wird man, z. B. wenn man als gewünschte Phänotyp eine RNase erhalten will, die hinter einem Adenosin schneidet, als Ausgangssequenz z. B. eine RNase, die hinter einem Guanosin schneidet, wählen (und nicht etwa eine Protease).

Umso ähnlicher die Ausgangsequenz in ihrem Phänotyp der gewünschten Eigenschaft jedoch ist, desto größer ist jedoch in der Regel auch die Hintergrundsaktivität in dem Test in Schritt c.) des Verfahrens. Vorteilhaft wird dieser Hintergrund vermieden, wenn die Ausgangssequenz nicht mehr in der Variantenbibliothek enthalten ist.

Bevorzugt wird die Variantenbibliothek daher so hergestellt, dass die Ausgangsvariante nicht mehr in der Variantenbibliothek enthalten ist. Dies kann zum Beispiel dadurch erreicht werden, dass in die Ausgangssequenz ein Stop-Codon eingefügt wird, welches durch das Einsetzen der mutierten Abschnitte in die Ausgangssequenz wieder entfernt wird. Somit wird sichergestellt, dass eventuell verschleppte Ausgangssequenzen durch das Stop-Codon physiologisch nicht aktiv sind und auf der anderen Seite, physiologisch aktive Varianten mutierte Bereiche enthalten müssen.

Im Gegensatz zu den nach dem Stand der Technik eingesetzten High-Throughput-Verfahren ermöglicht das erfindungsgemäße Verfahren ein Durchsuchen von um ein Vielfaches größerer Bibliotheken in einem Bruchteil der Zeit. Im Vergleich zu *in vivo* Selektionsverfahren ist das erfindungsgemäße Verfahren auch nicht limitiert auf bestimmte Enzymklassen bzw. bestimmte Enzymeigenschaften.

Im Schritt b.) wird die Variantenbibliothek in eine Anzahl Wo von Kompartimenten, die mindestens um einen Faktor 10, bevorzugt einen Faktor 100, kleiner ist, als die Anzahl der in der Varianten-Bibliothek enthaltenen Varianten, aufgeteilt.

Hierbei kann vor der Aufteilung die Überführung der Variantenbibliothek in einen Organismus erfolgen oder die Aufteilung auf Ebene der codierenden Sequenzen erfolgen. Die Aufteilung erfolgt so, dass jede Variante der Bibliothek mindestens einmal, bevorzugt genau einmal, vorkommt.

Die dann im Schritt c.) durchgeführte Produktion (Expression) der Biomoleküle erfolgt bevorzugt durch den Organismus oder durch *in vitro* Expressionssysteme (z.B. Zellextrakte).

Als Expressions-Organismus kommen alle in der Molekularbiologie standardmäßig zu Expression von Biomolekülen, wie Proteinen, verwendeten Organismen in Betracht, der Expressionsorganismus wird dabei in Abhängigkeit des zu exprimierenden Biomoleküls gewählt. Bevorzugte Expressionsorganismen sind balaerielle Zellen (z.B. *E. coli, B. subtilis*) oder eukaryontische Zellen (z.B. *S. cerevisiae,* Insektenzellen, Tumorzellen). Durch die Überführung der Variantenbibliothek in den Expressionsorganismus entstehen einzelne Klone des Expressionsorganismus. Dabei enthält ein Klon jeweils einen definierten Genotyp, d. h. eine Variante der für das Biomolekül codierenden Gensequenz. Ein Klon kann im Sinne der vorliegenden Erfindung auch durch eine alleinige codierende Sequenz definiert sein, d. h. einen definierten Genotyp ohne Expressionsorganismus.

Diese Überführung in einen Organismus geschieht durch die bekannten Methoden der Molekularbiologie für die Transformation von Gensequenzen in Expressionsorganismen und ist abhängig von dem verwendeten Expressionsorganismus. Eine bevorzugte Methode ist die Elektroporation.

Bevorzugt erfolgt die Aufteilung in Kompartimente sofort nach der Überführung der Variantenbibliothek in den Expressionsorganismus.

Die Anzahl Wo der Kompartimente beträgt zwischen $10^1$ und $10^4$ Kompartimenten und besonders bevorzugt zwischen 96 und 1536 Kompartimenten.

Die Bibliotheksgröße $B_0$ dividiert durch die Kompartiment-Anzahl Wo ergibt die KlonAnzahl $K_0$ pro Kompartiment, $K_0 \approx B_0 / W_0$.

Jedes Kompartiment enthält eine Teilbibliothek mit einer Anzahl von $K_0$ Varianten der für das Biomolekül codierenden Gensequenz.

Besonders bevorzugt erfolgt die Aufteilung in die Kompartimente einer Mikrotiter-bzw. Deepwellplatte mit Wo = 96 Kompartimenten

Im Schritt c.) erfolgt eine Vervielfältigung der Teilbibliotheken in den Kompartimenten durch Wachstum der Organismen oder Vervielfältigung der codierenden Sequenzen durch Matrizen-abhängige Enzyme bis zu einer Individuen-Anzahl $V_0$ pro Kompartiment und Produktion der katalytischen Biomoleküle durch die Expressionsorganismen oder zellfreie Expressionssysteme, wie z.B. *E. coli* Lysate, Reticulocyten-Lysate, *C. lucknowese* Lysate oder Insektenzellen-Lysate.

Eine Konservierung eines Anteils der Teilbibliothek erfolgt nun auf Organismen-Ebene oder der Ebene reiner codierender Sequenzen zum Zeitpunkt x unter Beibehaltung der Kompartiment-Zuordung.

Die Konservierung von Organismenkulturen erfolgt bevorzugt durch die Herstellung einer 1:1 Mischung aus Organismen-Kultur und Glycerol und Lagerung dieser Mischung unter Wachstumsinhibition bei -80°C. Eine Konservierung auf Ebene der codierenden Sequenzen erfolgt durch Abnahme eines Anteils der vervielfältigten Sequenzen und Lagerung, bevorzugt bei -20°C.

Eine Bestimmung der Individuen-Anzahl $V_0(x)$ der konservierten Teilbibliothek auf Organismen-Ebene erfolgt bevorzugt durch Messung der optischen Dichte OD einer flüssigen Organismen-Kultur und Korrelation mit der Individuen-Anzahl oder Überführung eines Aliquot dieser Kultur auf ein Festmedium und Auszählen der daraus resultierenden Kolonien. Die Bestimmung der Individuen-Anzahl $V_0(x)$ der konservierten Teilbibliothek auf Ebene codierender Sequenzen erfolgt bevorzugt durch Konzentrationsbestimmung mittels spektroskopischer Methoden.

Die Individuen-Anzahl $V_0(x)$ dividiert durch die Klonanzahl pro Kompartiment $K_0$ ergibt den Vervielfältigungsfaktor $F_0(x)$ pro Klon, $F_0(x) = V_0(x) / K_0$.

Im Schritt c.) des Verfahrens werden die in den einzelnen Kompartimenten enthaltenen Biomoleküle auf eine bestimmte Eigenschaft (Phänotyp), bevorzugt eine biokatalytische Aktivität, getestet.

In Schritt c.) erfolgt eine Vervielfältigung der Teilbibliothek in den Kompartimenten bis zu einer Individuen-Anzahl $V_0(x)$ zum Zeitpunkt x pro Kompartiment, wobei die Individuen-Anzahl $V_0(x)$ dividiert durch die Klonanzahl pro Kompartiment $K_0$ den Vervielfältigungsfaktor $F_0(x)$ pro Klon ergibt

Vor, während oder nach dem Wachstum der Organismen oder der Vervielfältigung der Genotypen erfolgt dabei die Produktion der Biomoleküle in den einzelnen Kompartimenten.

Bevorzugt erfolgt der Test auf eine biokatalytische Aktivität durch Inkubation der in dem Kompartiment enthaltenen oder aus diesen isolierten katalytisch aktiven Biomoleküle mit entsprechenden Substraten und Zuordnung von Aktivitätswerten zu den jeweiligen Kompartimenten. Kompartimente in denen der Aktivitätswert eine definierte Schwelle überschreitet, werden als positiv gewertet.

Da jedes Kompartiment mehr als einen Klon der Variantenbibliothek enthält, lässt sich aus dem beobachteten Phänotyp keine direkten Rückschlüsse auf den Genotyp machen, da der beobachtete Phänotyp durch die Summe der im Kompartiment enthaltenen Klone resultiert.

Obwohl im erfindungsgemäßen Verfahren daher Genotyp und Phänotyp entkoppelt sind lässt sich mit dem erfindungsgemäßen Verfahren der für die gewünschte Eigenschaft verantwortliche Klon, der zum Beispiel die gewünschte Enzymaktivität enthält, aus dem Gemisch der Klone wiederfinden und isolieren. Dass es möglich ist mit einem Screeningverfahren bei dem Genotyp und Phänotyp entkoppelt sind, den für die gewünschte Eigenschaft verantwortlichen Klon aus dem Gemisch der Klone wiederzuünden und zu isolieren ist für die Fachwelt überraschend, da alle bekannten Screeningverfahren auf der Kopplung von Genotyp und Phänotyp beruhen.

Den Klon mit der gewünschten Eigenschaft wiederzufinden wird durch die Schritte d.), und e.) des erfindungsgemäßen Verfahrens erreicht.

Im Schritt d.) des Verfahrens wird mindestens ein Kompartiment ausgewählt in dem Biomoleküle enthalten sind, welche die gewünschten Eigenschaften erfüllen.

Die in diesem Kompartiment enthaltene Teilbibliothek wird nun im Schritt e.) des Verfahrens entsprechend Schritt b.) erneut in Kompartimente aufgeteilt

Dazu wird die Teilbibliothek oder die entsprechende konservierte Teilbibliothek anhand des Faktor $F_0(x)$ so verdünnt, dass in einem gegebenen Volumen jeder in dem Kompartiment enthaltene Klon statistisch bis zu einer Anzahl $X_0 < W_1$ vorkommt. Dieses Volumen wird wiederum ohne vorherige Vervielfältigung auf neue Kompartimente der Anzahl $W_1$ aufgeteilt. Die neue Klonanzahl pro Kompartiment $K_1 \approx X_0 * K_0 / W_1$.

Nun werden die Schritte c.) bis e.) des Verfahrens solange wiederholt bis die Anzahl der Klone pro Kompartiment $K_n \leq 1$. Sobald $K_n \leq 1$ erreicht ist, ist der gesuchte Phänotyp einen einzelnen Genotyp zugeordnet.

Um den Verlust von Einzelklonen und somit Varianten der Bibliothek von Biomolekülen zu verhindern, wird der Schritt e.) bevorzugt in der Weise ausgeführt, dass in den ersten Durchläufen des Schritts e.) $1 < X_{n-1} < W_1$ gilt, bevorzugt $X_{n-1} = 3$ bis 5.

Der Schritt e.) wird bevorzugt solange wiederholt, bis der den gesuchten Phänotyp verursachende Klon innerhalb der neu-kompartimentierten Teilbibliothek zu finden ist. Hierbei ist bevorzugt im letzten Durchlauf des Schritts e.) $X_n < 1$. Vorzugsweise wird dazu bei der letzten Durchführung des Schrittes e.) die Teilbibliothek so verdünnt, dass maximal ein Klon pro Kompartiment zu finden ist und in vielen Kompartimenten kein Klon mehr enthalten ist. Damit gibt sich ein durchschnittlicher Wert von $X_n < 1$.

Im Schritt f.) werden die Schritte c.) bis e.) n-fach wiederholt bis in jedem Kompartiment nur noch maximal eine Variante ($K_n$<=1) der für das Biomolekül codierenden Gensequenz enthalten ist.

Die Anzahl der nötigen Wiederholungen n ist dabei abhängig von der Anzahl von Varianten ($B_0$) der in Schritt a.) eingesetzten Varianten-Bibliothek, der Anzahl der Kompartimente ($W_n$) in welche die Bibliotheken in Schritt b.) und e.) aufgeteilt werden und der Anzahl $X_n$, mit der ein einmal gefundener Klon im nächsten Zyklus wiedervorhanden ist. Die Anzahl der durchgeführten Wiederholungen n beträgt dabei bevorzugt bei konstantem $X_n = 1$ und konstantem $W_n$:

$$n = \log_{10}(B_0) - \log_{10}(W_n) \quad \text{oder} \quad n = (\log_{10}(B_0) - \log_{10}(W_n)) + 1,$$

wobei gegebenenfalls n auf die nächst höhere ganze Zahl aufgerundet wird.

Wird in Schritt a.) z. B. eine Bibliothek mit $B_0 = 10^6$ Varianten eingesetzt und werden die Teilbibliotheken in Schritt b.) und e.) jeweils mit $X_n = 1$ in $W_n = 96$ oder $W_n = 100$ Kompartimente aufgeteilt, sind n = 4 bis 5 Durchläufe der Schritte c.) bis e.) notwendig, um den Klon mit der gewünschten Aktivität wiederzufinden.

Anhand der nachfolgenden Ausführungsbeispiele wird die Erfindung näher erläutert:

Ausführungsbeispiel 1 beschreibt als Beispiel die Selektion von aktiver RNase T1 aus einer Variantenbibliothek von inaktiven Varianten von RNase T1.

Ausführungsbeispiel 2 beschreibt als Beispiel die Selektion einer nach Adenosin spaltenden RNase T1 aus einer Bibliothek von RNase T1-Varianten

## Ausführungsbeispiel 1

1. Klonierung der Gene für RNase T1 Wildtyp und His92Ala

Mit den beiden Primern A2Vo_BspHI (SEQ_ID No. 1) und A2Hi_PstI (SEQ_ID No. 2) (beide IBA Göttingen) werden die für RNase T1 Wildtyp (SEQ_ID No. 3) und die RNase T1 Variante His92Ala (SEQ_ID No. 4) codierenden Gene inklusive des Signalpeptides für eine periplasmatische Expression aus dem jeweiligen Ursprungsvektoren pA2T1 (SEQ_ID No. 5) und pA2T1_H92A (SEQ_ID No. 5, in dem SEQ_ID No. 3 durch SEQ_ID No. 4 ersetzt ist) durch eine PCR unter den nachfolgenden Bedingungen amplifiziert.

1.1 PCR:

| | | | |
|---|---|---|---|
| PCR-Ansatz: | 10 µl | 10x VENT-Puffer (NEB, Beverly, USA) | |
| | 2 µl | dNTPs (je 10 mmol/Liter) | |
| | 100 pmol | Primer A2Vo_BspHI | (SEQ_ID No. 1) |
| | 100 pmol | Primer A2Hi_PstI | (SEQ_ID No. 2) |
| | 1 µl | Ursprungsvektor (20 ng) | (SEQ_ID No. 5) |
| | 2 U | VENT-Polymerase (NEB) | |
| | ad 100 µl | $H_2O$ dest. | |

| | | | |
|---|---|---|---|
| Temperaturprofil der PCR: | 2 min / 94 °C | | |
| | 1. 45 sec / 94 °C (Denaturierung) | 25 x | |
| | 2. 45 sec / 57 °C (Anlagerung) | | |
| | 3. 30 sec / 72 °C (Elongation) | | |
| | 2 min / 72°C | | |

Die resultierenden PCR-Produkte werden mittel des QIAquick PCR-Reinigungs-Kit (Qiagen, Hilden) nach Herstellervorschrift gereinigt.

1.2 Restriktionsverdau:

Zur Klonierung der Gene in den Expressionsvektor pETBlue-2 (SEQ_ID No. 6) werden die PCR-Produkte und der Vektor mittels Restriktionsendonucleasen BspHI und PstI bzw. NcoI und PstI (alle MBI Fermentas, Vilnius, Litauen) wie folgt inkubiert:

Restriktionsverdau-Ansätze:

| PCR-Produkte: | | Vektor: | |
|---|---|---|---|
| 2 µg | PCR-Produkt | 4 µg | pETBlue-2 |
| 2 µl | 10x Puffer $O^+$ (MBI) | 2 µl | 10x Puffer $Y^+$ (MBI) |
| 10 U | BspHI | 10 U | NcoI |
| 10 U | PstI | 10 U | PstI |
| ad 20 µl | $H_2O$ dest. | ad 20 µl | $H_2O$ dest. |

Die Restriktionsverdau-Ansätze werden 2 h bei 37 °C inkubiert. Zu dem "Vektor-Ansatz" wird anschließend zur Dephosphorylierung 1 U SAP (MBI Fermentas, Vilnius, Litauen) hinzu gegeben und für weitere 30 min bei 37 °C inkubiert. Anschließend werden die Enzyme für 20 min bei 80 °C inaktiviert. Daraufhin werden die Produkte mittels des QIAquick PCR-Reinigungs-Kit (Qiagen, Hilden) aufgereinigt.

1.3 Ligation. Transformation in *E. coli* und Plasmid-Reisolation

Die Velctor-DNA und das PCR-Produkt werden durch die Inkubation mit T4-DNA-Ligase wie folgt miteinander verbunden:

| | | |
|---|---|---|
| Ligase-Ansatz: | 200 fmol | Vektor-DNA |
| | 600 fmol | PCR-Produkt |
| | 3 µl | 10x Ligase-Puffer (MBI) |
| | 1 µl | T4-DNA-Ligase |
| | ad 30 µl | $H_2O$ dest. |

Die Ansätze werden 8 h bei 16 °C inkubiert und anschließend wurde das Enzym durch 10-minütige Inkubation bei 65 °C inaktiviert. 1 µl dieses Ansatzes wurde direkt zur Transformation kommerziell erhältlicher kompetenter ElectroTen-Zellen (Stratagene, La Jolla, USA) mittels Elektroporation eingesetzt. Die elektroporierten Zellen wurden auf Festagar-Platten mit Ampicillin ausplatiert und über Nacht bei 37 °C kultiviert. Ausgehend von einer resultierenden Einzelkolonie wird das fertige Plasmid mittels des Plasmid-Reinigungskits QIAprep Minipräparations-Kit (Qiagen, Hilden) nach Herstellervorschrift reisoliert.

1.4 Herstellung einer Plasmid-Mischung als RNAse T1-Testbibliothek:

Als Resultat aus den vorangegangenen Schritten werden die beiden Plasmide pETBlue-RNaseT1-Wildtyp und pETBlue-RNaseT1-His92Ala erhalten.
Zur Herstellung einer Testbibliothek werden die Plasmide wie folgt gemischt:

1 pg pETBlue-RNaseT1-Wildtyp wird mit 1 µg pETBlue-RNaseT1-His92Ala gemischt. Dadurch erhält man ein Verhältnis von 1 : 1.000.000 aus RNase T1 Wildtyp (aktiv) und der Variante His92Ala (inaktiv).

1.5 Herstellung des Expressionsstammes:

Für die Expression der RNase T1-Testbibiliothek wird ein *E. coli* Stamm benötigt, bei dem die RNase I ausgeschaltet ist. Entsprechende Stämme, wie z. B. AT9 (rna-19 λ-gdhA2 relA1 spoT1 metB1) sind über das E. coli Genetic Stock Center New Haven, USA verfügbar. Der im Beispiel verwendete Expressionsvektor pETBlue-2 benötigt zusätzlich die T7-RNA-Polymerase für die Expression, welche in *E. coli* nicht vorhanden ist. Mit dem kommerziell erhältlichen λDE3-Lysogenisierungs-Kit (Novagen, Madison, USA) wird nach Herstellervorschrift das T7-RNA-Polymerase codierenden Gen in den *E.* coli Stamm AT9 eingeführt. Hierdurch erhält man einen *E. coli* Stamm, der sich durch die Abwesenheit von RNase I und das Vorhandensein der T7-RNA-Polymerase (DE3) auszeichnet. Von diesem Stamm wurden mittels Standard-Molekularbiologie-Methoden elektrokompetente Zellen hergestellt und bei -80 °C gelagert.

1.6 Transformation des Expressionsstammes mit der Testbibliothek:

In den wie oben beschrieben hergestellten Expressionsstamm wird 1 ng der Plasmid-Mischung als Testbibliothek mittels Elektroporation transformiert und die resultierenden Zellen nach 1-stündigem Wachstum bei 37 °C in 10 ml Flüssigmedium (LB-Medium: 10 g Trypton, 5 g Hefe-Extrakt (alles Becton Dickinson, Heidelberg), 10 g NaCl (Sigma, Deisenhofen)) mit Ampicillin aufgenommen

Die so erhaltene Vorkultur wird sofort auf eine 96-Well Mikrotiterplatte (MTP) aufgeteilt (je 100 µl pro well) und über Nacht bei 30 °C und 800 Upm inkubiert.

Durch die Transformationen mittels Elektroporation werden ca. 3 Millionen transformierte Klone erhalten.

1.7 Wachstum der Hauptkultur und Expression von RNase T1

Eine 96er Deepwellplatte (DWP) wird mit jeweils 1,5 ml Flüssigmedium mit Ampicillin pro Well befüllt. Das Medium wird mit jeweils 50 µl aus der Vorkultur-MTP beimpft und die DWP bei 37 °C und 800 Upm kultiviert. Beim Erreichen einer optischen Dichte $OD_{600}$ der Kulturen von $OD_{600}$ = 1,0 werden die Kulturen mit 1 mmol/Liter IPTG induziert. Anschließend wird die Platte für weitere 4 h bei 37 °C und 800 Upm inkubiert.

1.8 Präparation der Protein-Proben

Durch das Signalpeptid ompA werden die exprimierten RNase T1-Moleküle in den periplasmatischen Raum des Expressionsbakteriums geleitet. Durch einen osmotischen Schock können die Proteine sehr leicht präpariert werden. Die Reinigungsprozedur umfasst dabei folgende Schritte:

- Sammeln der Zellen durch Zentrifugation bei 4000 Upm, 4 °C für 5 min,
- Abschütten des Medien-Überstandes,
- Resuspension des Bakterienpellets in jeweils 25 µl Puffer A (50 mmol/Liter Tris/HCl, pH 7,5, 10 mmol/Liter EDTA, 15 % Saccharose w/v),
- Inkubation auf Eis für 30 min,
- Zugabe von jeweils 125 µl Puffer B (50 mmol/Liter Tris/HCl, pH 7,5, 10 mmol/Liter EDTA),
- Zentrifugation bei 4000 Upm, 4 °C, für 20 min,
- Abnahme des Überstandes und Überführung in eine MTP (Periplasma),
- Aufbewahren der Bakterienpellets.

1.9 Herstellung des Substrates für RNase T1

Als Substrat (Sub_G) dient ein doppelsträngiges DNA-Molekül mit zentralem einzelsträngigen Bereich, welcher einen Guanosin-RNA-Baustein (rG) als Angriffspunkt für das Enzym enthält. Die Enden dieses Substrates sind mit unterschiedlichen Farbstoffen für den roten (Cy5 am 5'-Ende) und den grünen (RhG am 3'-Ende) Spektralbereich markiert. Um ein Ausbleichen der markierten Substrate zu verhindern, werden die entsprechenden Lösungen und Inkubationsansätze stets vor Licht geschützt. Die Puffer und Ansätze werden mit DEPC-behandeltem Wasser hergestellt. Das Substrat setzt sich aus den folgenden 3 Oligonucleotiden (IBA Göttingen) zusammen:

1. Sub_G:

   5'-Cy5-CCATACCAGCCAGCCACAArGCAAGCCACCGAAGCACAGATA-RhG-3' (SEQ_ID No. 10)

2. T1_Sub_Li:

   5'-GTGGCTGGCTGGTATGGA-3' (SEQ_ID No. 7)

3. T1_Sub_Re:

   5'-TATCTGTGCTTCGGTGGC-3' (SEQ_ID No. 8)

Durch die nachfolgend beschriebene Hybridisierung werden die 3 Bestandteile wie folgt zu einem doppelsträngigen Substrat aneinander angelagert:

| Hybridisierungs-Ansatz: | Hybridisierungs-Programm: |
|---|---|
| 1000 pmol Sub_G | 1. 10s 94°C; |
| 1200 pmol T1_Sub_Li | 2. Abkühlen auf 25 °C mit 0,1 °C/s |
| 1200 pmol T1_Sub_Re | 3. 4 °C |
| 20 µl MES (1 mol/Liter, pH 6,0) | |

(fortgesetzt)

| Hybridisierungs-Ansatz: | Hybridisierungs-Programm: |
|---|---|
| ad 1000 µl DEPC-$H_2O$ | |

1.10 Inkubation der Protein-Proben mit dem Substrat

In einer MTP werden jeweils 10 µl des doppelsträngigen Substrates pro Well vorgelegt. Dazu werden jeweils 10 µl der aus dem Periplasma isolierten Protein-Proben hinzugegeben, die MTP luftdicht verschlossen und dunkel für 24 h bei 37 °C inkubiert. Anschließend werden jeweils 5 µl dieser Ansätze in eine MTP mit Glasboden überführt und mit jeweils 250 µl Puffer C (100 mmol/Liter MES, pH 6,0, 100 mmol/Liter NaCl, 2 mmol/Liter EDTA) gemischt.

1.11 Aktivitätsbestimmung

Zur Bestimmung der Enzymaktivität wird die Platte mit Glasboden, in die die Inkubationsansätze wie unter 1.10 beschrieben überführt wurden, am Fluoreszenz-Korrelations-Spektroskop ConfoCor 2 (Evotec Biosystems, Hamburg und Carl Zeiss Microscopy, Jena) vermessen. Die Auswertung der Daten erfolgt mit der ConfoCor 2-Software (Version 2.5).

Für die Messungen wird ein Argon-Laser (1 = 488 nm) zur Anregung von RhG in Kombination mit einem Helium/Neon-Laser (1 = 633 nm) für Cy5 eingesetzt. Das FCS-Messvolumen in den Kavitäten wurde 200 µm über der Glasbodenoberfläche justiert. Die Messungen erfolgen für 20 s pro Well.

Durch eine Kreuzkorrelationsanalyse der erhaltenen Daten kann auf eine eventuelle Spaltung des Substrates geschlossen werden. Eine Spaltung des Substrates durch RNase T1 führt zu einer Entkopplung der beiden Fluoreszenzfarbstoffe und somit zum Verlust des Kreuzkowelationssignales. Ungeschnittene Substrat-Moleküle tragen hingegen beide Farbstoffe und liefern ein hohes Signal.

Durch die Aufteilung der durch die Transformation erhaltenen 3 Millionen Klone und das Mischungsverhältnis zwischen aktiver RNase T1 Wildtyp und inaktiver RNase T1 His92Ala von 1 : 1.000.000 sollten theoretisch 3 Wells mit Aktivität durch die Messungen detektierbar sein. Statistische Abweichungen zwischen 1 bis 5 Wells mit Aktivität sind jedoch möglich.

**Fig. 1** zeigt die so erhaltenen Messdaten für ein nach Punkt 1 bis 1.11 hergestellte RNase T1-Testbibliothek bestehend aus 3 Millionen Klonen auf einer Platte mit einem Mischungsverhältnis von RNase T1 Wildtyp und RNase T1-His92Ala von 1:1.000.000. Die RNase T1-Aktivität wurde wie oben beschrieben mittels Kreuzkorrelationsanalyse detektiert. Für eine bessere Übersicht wurde eine reziproke Darstellung gewählt, d.h. hohe Peaks bedeuten ein niedriges Signal und niedrige Peaks ein hohes Signal. Fig. 1 zeigt 2 deutliche Peaks, die durch einen Verlust des Kreuzkorrelationssignals hervorgerufen werden. Diese beiden Peaks zeigen, dass in dem Experiment eine RNase T1-Aktivität in zwei von 96 Wells sicher vorhanden war.

2. Reisolation der Teilbibliothek

In der nach Punkt 1 erhalten Platte mit den aufbewahrten Bakterienpellets aus der Proteinpräparation wird in einem der Wells, welche in der Aktivitätsbestimmung (Punkt 1.11) eine RNase T1-Aktivität gezeigt hat, eine Plasmidpräparation mittels dem QIAprep Minipräparations-Kit (Qiagen, Hilden) durchgeführt.

Durch die ursprüngliche Aufteilung von 3 Millionen Klonen auf die Platte ergab sich eine Anzahl von 3.000.000 / 96 = 31.250 unterschiedlichen Klonen pro Well. Es besteht somit in der isolierten Teilbibliothek ein Mischungsverhältnis von RNase T1 Wildtyp zu RNase T1 His92Ala von 1 : 32.250.

2.1 Weitere Vereinzelung

Durch eine Transformation von verschiedenen Aliquots der so erhaltenen Teilbibliothek analog der vorgehensweiße von Punkt 1.6 wurde die Menge von Plasmid-DNA bestimmt, welche notwendig ist, um nun ca. 100.000 transformierte Klone mittels Elektroporation zu erhalten.

Anschließend wurde die bestimmte Menge der Teilbibliothek in den Expressionsstamm transformiert und das gleiche Verfahren wie für die ursprüngliche Testbibliothek durchlaufen. Da ca. 100.000 Klone aufgeteilt wurden und das neue Mischungsverhältnis 1 : 32.250 betrug, waren wieder theoretisch 3 Wells mit detektierbarer Aktivität zu erwarten.

Die Plasmide wurden wiederum in einer der Wells mit Aktivität aus dem Bakterienpellet reisoliert. Das Mischungsverhältnis in dieser erneut angereicherten Teilbibliothek war nun 100.000 / 96 = 1050.

Eine weitere Wiederholung des dargestellten Schemas mit einer Aufteilung von jetzt ca. 3000 Klonen ergab eine nochmals angereicherte Teilbibliothek mit einem Mischungsverhältnis von 3000 / 96 = 31.

Indem von dieser letzten Teilbibliothek 96 Klone auf eine MTP aufgeteilt wurden, resultierten daraus 3 Wells mit Aktivität. Da diese Aktivitäten nun jeweils von einem vereinzelten Klon verursacht wurden, konnte diesem die Aktivität von RNase T1 Wildtyp direkt zugeordnet werden.

**Ausführungsbeispiel 2**

Wildtyp RNase T1 spaltet RNA hoch spezifisch nach Guanosin-Resten. Zielstellung dieses Ausführungsbeispiels ist es RNase T1 Varianten, die RNA nach Adenosin-Resten spalten können, zu erhalten. Hierfür wurde eine RNase T1-Bibliothek erstellt und nach entsprechenden Varianten durchmustert.

1. Design der Bibliothek

Der zu mutierende Bereich des Guanin-Bindungsloops 1 umfasst die Aminosäuren 41 bis 57 von RNase T1 Wildtyp (SEQ_ID No. 3). Die Loop 1-DNA-Sequenz wird durch ein entsprechend synthetisiertes Mutagenese-Oligodesoxynucleotid Loop1_32 (SEQ_ID No. 9) so mutiert, dass je Variante 3 bis 4 der 17 Aminosäuren zufällig durch andere ersetzt werden. Dazu wird folgende Sequenz synthetisiert:

5'-GTAGGATCCAATTCTTACCCACAC aay tax aax aax tax gay ggz ttz gaz
ttx tcz gty agx tcz ccx tax tax GAATGGCCTATCCTCTCGAGCGG-3'

in der "n" (A, G, C oder T -"any") und "b" (G, C oder T - nicht A) aus SEQ_ID No. 9 wie folgt näher definiert sind:

| | |
|---|---|
| a = 86 % A 6 % C 4 % G | x = c' = 88 % C 6 % G 6 % T |
| c = 86 % C 6 % A 4 % G | y = g' = 82 % G 11 % C 7 % T |
| g = 79 % G 8%A 8 % C | |
| t = 79 % T 8 % A 8 % C | z = t' = 82 % T 11% C 7 % G |

mit A = Adenin, C = Cytosin, G = Guanin, T = Thymin.

Das Oligonucleotid Loop1_32 (IBA, Göttingen, Germany) wird anschließend in einer PCR direkt als Primer (unter Punkt 3.1) eingesetzt.

2. Herstellung des Vektors für das Screening

In den Vektor pETBlue-2 (SEQ_ID No. 6) wird, wie in Ausführungsbeispiel 1 (Punkt 1.1. - 1.3.) beschrieben, das Gen für RNase T1 Wildtyp (SEQ_ID No. 3) inklusive des Signalpeptides für eine periplasmatische Expression kloniert und der Vektor pETBlue-RNaseT1-Wildtyp erhalten.

Anschließend wird der Vektor pETBlue-RNaseT1-Wildtyp mit PvuII und SspI (beide MBI Fermentas, Vilnius, Litauen) verdaut:

Ansatz:

| | |
|---|---|
| 4 µg | pETBlue-2 |
| 2 µl | 10x Puffer G (MBI) |
| 10 U | SspI |
| 10 U | PvuII |
| ad 20 µl | $H_2O$ dest. |

Der Restriktionsverdau-Ansatz wird 2 h bei 37 °C inkubiert. Anschließend werden die Enzyme für 20 min bei 80 °C inaktiviert. Die Produkte werden auf einem 0,8 %igen Agarose-Gel getrennt und die Produktbande bei 2498 bp aus dem Gel ausgeschnitten. Die DNA wird daraufhin mittels des QIAquick Gel-Extraktions-Kit (Qiagen, Hilden) reisoliert. 200 fmol des isolierten Fragmentes werden in einer Ligation rezirkularisiert:

Ansatz: 200 fmol Fragment

(fortgesetzt)

| | |
|---|---|
| 2 µl | 10x Ligase-Puffer (MBI) |
| 2 µl | 50 % PEG (MBI) |
| 1 µl | T4-DNA-Ligase |
| ad 20 µl | $H_2O$ dest. |

Der Ansatz wird 8 h bei 16 °C inkubiert und anschließend wird das Enzym durch 10-minütige Inkubation bei 65 °C inaktiviert. 1 µl dieses Ansatzes wird direkt zur Transformation kommerziell erhältlicher kompetenter ElectroTen-Zellen (Stratagene, La Jolla, USA) mittels Elektroporation eingesetzt. Die elektroporierten Zellen wurden auf Festagar-Platten mit Ampicillin ausplatiert und über Nacht bei 37 °C kultiviert. Ausgehend von einer resultierenden Einzelkolonie wird das fertige Plasmid mittels des Plasmid-Reinigungskits QIAprep Minipräparations-Kit (Qiagen, Hilden) nach Herstellervorschrift reisoliert. Das so erhaltene Plasmid wird als pETMini_RNaseT1_ Wildtyp bezeichnet.

3. Klonierung der Bibliothek RNase T1-Loop1

Mit den beiden Primern Loop1_32 (SEQ_ID No. 9) und A2Hi_PstI (SEQ_ID No. 2) (beide IBA Göttingen) wird ein Teil des für RNase T1 Wildtyp (SEQ ID No. 3) aus dem Ursprungsvektoren pA2T1 (SEQ ID No. 5) durch eine PCR unter den nachfolgenden Bedingungen aniplifizieil.

3.1 PCR:

| | | | |
|---|---|---|---|
| PCR-Ansatz: | 10 µl | 10x Taq-Puffer (MBI Fermentas, Vilnius, Litauen) | |
| | 2 µl | dNTPs (je 10 mmol/Liter) | |
| | 100 mol | Primer Loop1_32 | (wie aus Punkt 1) |
| | 100 prmol | Primer A2Hi_PstI | (SEQ_ID No. 2) |
| | 1 µl | Ursprungsvektor (20 ng) | (SEQ_ID No. 5) |
| | 2 U | Taq-Polymerase (MBI) | |
| | ad 100 µl | $H_2O$ dest. | |

Temperaturprofil der PCR: 2 min / 94 °C

| | | |
|---|---|---|
| 1. | 45 sec / 94 °C (Denaturierung) | 30 x |
| 2. | 45 sec / 57 °C (Anlagerung) | |
| 3. | 30 sec / 72 °C (Elongation) | |

2 min / 72 °C

Die resultierenden PCR-Produkte werden mittel des QIAquick PCR-Reinigungs-Kit (Qiagen, Hilden) nach Herstellervorschrift gereinigt.

3.2 Restriktionsverdau:

Zur Klonierung der Bibliothek in den Expressionsvektor pETMini_RNaseT1_Wildtyp werden das PCR-Produkt und der Vektor mittels Restriktionsendonucleasen BamHI und PstI (alle MBI Fermentas, Vilnius, Litauen) wie folgt inkubiert:

Restriktionsverdau-Ansätze:

| PCR-Produkte: | | Vektor: | |
|---|---|---|---|
| 2 µg | PCR-Produkt | 4 µg | pETMini_RNaseT1_Wildtyp |

(fortgesetzt)

| PCR-Produkte: | | Vektor: | |
|---|---|---|---|
| 2 µl | 10x Puffer $G^+$ (MBI) | 2 µl | 10x Puffer $G^+$ (MBI) |
| 10U | BamHI | 10U | BamHI |
| 10U | PstI | 10U | PstI |
| ad 20 µl | $H_2O$ dest. | ad 20 µl | $H_2O$ dest. |

Die Restriktionsverdau-Ansätze werden 2 h bei 37 °C inkubiert. Zu dem "Vektor-Ansatz" wird anschließend zur Dephosphorylierung 1 U SAP (MBI Fermentas, Vilnius, Litauen) hinzu gegeben und für weitere 30 min bei 37 °C inkubiert. Anschließend werden die Enzyme für 20 min bei 80 °C inaktiviert. Die Produkte werden auf einem 0,8 %igen Agarose-Gel getrennt und bei dem Vektoransatz die Produktbande bei 2608 bp und bei dem PCR-Ansatz die Produktbande bei 259 bp aus dem Gel ausgeschnitten. Die DNA wird daraufhin mittels des QIAquick Gel-Extraktions-Kit (Qiagen, Hilden) aus den Gel-Stücken reisoliert.

3.3 Ligation. Transformation in *E. coli* und Plasmid-Reisolation

Die Vektor-DNA und das PCR-Produkt werden durch die Inkubation mit T4-DNA-Ligase wie folgt miteinander verbunden:

| | | |
|---|---|---|
| Ligase-Ansatz: | 200 fmol | Vektor-DNA |
| | 600 fmol | PCR-Produkt |
| | 3 µl | 10x Ligase-Puffer (MAI) |
| | 1 µl | T4-DNA-Ligase |
| | ad 30 µl | $H_2O$ dest. |

Die Ansätze werden 8 h bei 16 °C inkubiert und anschließend wurde das Enzym durch 10-minütige Inkubation bei 65 °C inaktiviert. Die Enzyme werden mit 2-maligen Ausschütteln mit Phenol/Chloroform aus der Lösung entfernt und die erhaltene wässrige Lösung durch Zugabe des 2,5-fachen Volumens Ethanol durch Inkubation für . 1 h bei -20 °C gefällt. Der Ansatz wird anschließend 30 min bei 13000 Upm, 4 °C zentrifugiert und das Pellet in 50 µl 70 %igem Ethanol gewaschen. Nach einer weiteren 15 minütigen Zentrifugation bei 13000 Upm, 4 °C wird der Ethanol abgenommen und das DNA-Pellet getrocknet. Anschließend wird die DNA in 3 µl $H_2O$ dest. aufgenommen direkt zur Transformation kommerziell erhältlicher kompetenter ElectroTen-Zellen (Stratagene, La Jolla, USA) mittels Elektroporation eingesetzt. Von den elektroporierten Zellen werden 10 µl auf Festagar-Platten mit Ampicillin ausplatiert und über Nacht bei 37 °C kultiviert. Der Rest der elektroporierten Zellen wird direkt in 100 ml Flüssigmedium (LB-Medium: 10 g Trypton, 5 g Hefe-Extrakt (alles Becton Dickinson, Heidelberg), 10 g NaCl (Sigma, Deisenhofen)) mit Ampicillin verdünnt und ebenfalls über Nacht inkubiert. Die Kolonien auf der Festagar-Platte werden ausgezählt und aus dem Wert die Größe der Gesamtbibliothek bestimmt. Ausgehend von 5 ml der in Flüssigkultur gewachsenen Klonmischung wird die fertige Plasmid-Bibliothek mittels des Plasmid-Reinigungskits QIAprep Minipräparations-Kit (Qiagen, Hilden) nach Herstellervorschrift reisoliert. Als Resultat erhält man eine Bibliothek aus bis zu $10^7$ verschiedenen RNaseT1_Loop1_Varianten: pETMini_RNaseT1_L1.

3.4 Herstellung des Expressionsstammes:

Für die Expression der RNase T1-Testbibiliothek wird ein *E. coli* Stamm benötigt, bei dem die RNase I ausgeschaltet ist. Entsprechende Stämme, wie z. B. AT9 (rna-19 $\lambda^-$gdhA2 relA1 spoT1 metB1) sind über das E. coli Genetic Stock Center New Haven, USA verfügbar. Der im Beispiel verwendete Expressionsvektor pETBlue-2 benötigt zusätzlich die T7-RNA-Polymerase für die Expression, welche in *E. coli* nicht vorhanden ist. Mit dem kommerziell erhältlichen λDE3-Lysogenisierungs-Kit (Novagen, Madison, USA) wird nach Herstellervorschrift das T7-RNA-Polymerase codierenden Gen in den *E.* coli Stamm AT9 eingeführt. Hierdurch erhält man einen *E. coli* Stamm, der sich durch die Abwesenheit von RNase I und das Vorhandensein der T7-RNA-Polymerase (DE3) auszeichnet. Von diesem Stamm wurden mittels Standard-Molekularbiologie-Methoden elektrokompetente Zellen hergestellt und bei -80 °C gelagert.

3.5 Transformation des Expressionsstammes mit der Bibliothek:

In den wie oben beschrieben hergestellten Expressionsstamm wird 1 ng der Bibliothek pETMini_RNaseT1_L1 mittels Elektroporation transformiert und die resultierenden Zellen nach 1-stündigem Wachstum bei 37 °C in 200 ml Flüssigmedium (LB-Medium: 10 g Trypton, 5 g Hefe-Extrakt (alles Becton Dickinson, Heidelberg), 10 g NaCl (Sigma, Deisenhofen)) mit Ampicillin aufgenommen. 10 ml der so erhaltenen Vorkultur werden sofort auf eine 96-Well Mikrotiterplatte (MTP) aufgeteilt (je 100 µl pro well) und über Nacht bei 30 °C und 800 Upm inkubiert. Dadurch werden ca. 150.000 Klone auf der MTP erhalten.

3.6 W achstum der Hauptkultur und Expression von RNase T1

Eine 96er Deepwellplatte (DWP) wird mit jeweils 1,5 ml Flüssigmedium mit Ampicillin pro Well befüllt. Das Medium wird mit jeweils 50 µl aus der Vorkultur-MTP beimpft und die DWP bei 37 °C und 800 Upm kultiviert. Beim Erreichen einer optischen Dichte $OD_{600}$ der Kulturen von $OD_{600}$ = 1,0 werden die Kulturen mit 1 mmol/Liter IPTG induziert. Anschließend wird die Platte für weitere 4 h bei 37 °C und 800 Upm inkubiert.

3.7 Präparation der Protein-Proben

Durch das Signalpeptid ompA werden die exprimierten RNase T1 - Moleküle in den periplasmatischen Raum des Expressionsbakteriums geleitet. Durch einen osmotischen Schock können die Proteine sehr leicht präpariert werden. Die Reinigungsprozedur umfasst dabei folgende Schritte:

- Sammeln der Zellen durch Zentrifugation bei 4000 Upm, 4 °C für 5 min
- Abschütten des Medien-Überstandes
- Resuspension des Bakterienpellets in jeweils 25 µl Puffer A (50 mmol/Liter Tris/HCl, pH 7,5, 10 mmol/Liter EDTA, 15 % Saccharose w/v)
- Inkubation auf Eis für 30 min
- Zugabe von jeweils 125 µl Puffer B (50 mmol/Liter Tris/HCl, pH 7,5, 10 mmol/Liter EDTA)
- Zentrifugation bei 4000 Upm, 4 °C, für 20 min
- Abnahme des Überstandes und Überführung in eine MTP (Periplasma)
- Aufbewahren der Bakterienpellets

3.8 Herstellung des Substrates für RNase T1

Als Substrat (Sub_A) dient ein doppelsträngiges DNA-Molekül mit zentralem einzelsträngigen Bereich, welcher nun einen Adenosin-RNA-Baustein (rA) als Angriffspunkt für das Enzym enthält. Die Enden dieses Substrates sind mit unterschiedlichen Farbstoffen für den roten (Cy5 am 5'-Ende) und den grünen (RhG - am 3'-Ende) Spektralbereich markiert. Um ein Ausbleichen der markierten Substrate zu verhindern, werden die entsprechenden Lösungen und Inkubationsansätze stets vor Licht geschützt. Die Puffer und Ansätze werden mit DEPC-behandeltem Wasser hergestellt. Das Substrat setzt sich aus den folgenden 3 Oligonucleotiden (IBA Göttingen) zusammen:

1. Sub_A:

   5'-Cy5-CCATACCAGCCAGCCACAArACAAGCCACCGAAGCACAGATA-RhG-3' (SEQ ID No. 11)

2. T1_Sub_Li:

   5'-GTGGCTGGCTGGTATGGA-3' (SEQ_ID No. 7)

3. T1_Sub_Re:

   5'-TATCTGTGCTTCGGTGGC-3' (SEQ_ID No. 8)

Durch die nachfolgend beschriebene Hybridisierung werden die 3 Bestandteile wie folgt zu einem doppelsträngigen Substrat aneinander angelagert:

| Hybridisierungs-Ansatz: | Hybridisierungs-Programm: |
|---|---|
| 1000 pmol Sub_A | 1. 10 s 94°C; |
| 1200 pmol T1_Sub_Li | 2. Abkühlen auf 25 °C mit 0,1 °C/s |
| 1200 pmol T1_Sub_Re | 3. 4 °C |

(fortgesetzt)

| Hybridisierungs-Ansatz: | Hybridisierungs-Programm: |
|---|---|
| 20 µl MES (1 mol/Liter, pH 6,0) | |
| ad 1000 µl DEPC-$H_2O$ | |

3.9 Inkubation der Protein-Proben mit dem Substrat

In einer MTP werden jeweils 10 µl des doppelsträngigen Substrates pro Well vorgelegt. Dazu werden jeweils 10 µl der aus dem Periplasma isolierten Protein-Proben hinzugegeben, die MTP luftdicht verschlossen und dunkel für 24 h bei 37 °C inkubiert. Anschließend werden jeweils 5 µl dieser Ansätze in eine MTP mit Glasboden überführt und mit jeweils 250 µl Puffer C (100 mmol/Liter MES, pH 6,0, 100 mmol/Liter NaCl, 2 mmol/Liter EDTA) gemischt.

3.10 Aktivitätsbestimmung

Zur Bestimmung der Enzymaktivität wird die Platte mit Glasboden, in die die Inkubationsansätze wie unter 1.10 beschrieben überführt wurden, am Fluoreszenz-Korrelations-Spektroskop ConfoCor 2 (Evotec Biosystems, Hamburg und Carl Zeiss Microscopy, Jena) vermessen. Die Auswertung der Daten erfolgt mit der ConfoCor 2-Software (Version 2.5).

Für die Messungen wird ein Argon-Laser (1 = 488 nm) zur Anregung von RhG in Kombination mit einem Helium/Neon-Laser (1 = 633 nm) für Cy5 eingesetzt. Das FCS-Messvolumen in den Kavitäten wurde 200 µm über der Glasbodenoberfläche justiert. Die Messungen erfolgen für 20 s pro Well.

Durch eine Kreuzkorrelationsanalyse der erhaltenen Daten kann auf eine eventuelle Spaltung des Substrates geschlossen werden. Eine Spaltung des Substrates durch RNase T1 führt zu einer Entkopplung der beiden Fluoreszenzfarbstoffe und somit zum Verlust des Kreuzkorrelationssignales. Ungeschnittene Substrat-Moleküle tragen hingegen beide Farbstoffe und liefern ein hohes Signal.

**Fig. 2** zeigt die so erhaltenen Messdaten für ein nach Ausführungsbeispiel 2 hergestellte RNase T1-Loop1-Bibliothek bestehend aus 150.000 Klonen auf einer Platte. Die RNase T1-Aktivität wurde wie oben beschrieben mittels Kreuzkorrelationsanalyse detektiert. Für eine bessere Übersicht wurde eine reziproke Darstellung gewählt, d.h. hohe Peaks bedeuten ein niedriges Signal und niedrige Peaks ein hohes Signal. Fig. 2 zeigt einen sehr deutlichen Peak, die durch einen Verlust des Kreuzkorrelationssignales hervorgerufen wird. Der Peak zeigt, dass in dem Experiment eine RNase T1-Aktivität, welche nun ein Substrat nach A spalten kann, in einem von 96 Wells vorhanden war.

4. Reisolation der Teilbibliothek

In der nach Ausführungsbeispiel 2 (Punkte 1. - 3.10.) erhaltenen Platte mit den aufbewahrten Bakterienpellets aus der Proteinpräparation wird in dem Well, welche in der Aktivitätsbestimmung (3.10) eine RNase T1-Aktivität nach Adenosin gezeigt hat, eine Plasmidpräparation mittels dem QIAprep Minipräparations-Kit (Qiagen, Hilden) durchgeführt.

Durch die ursprüngliche Aufteilung von 150.000 Klonen auf die Platte ergab sich eine Anzahl von 150.000 / 96 = 1563 unterschiedlichen Klonen pro Well.

5.1 Weitere Vereinzelungen - 1. Schritt

Durch eine Transformation von verschiedenen Aliquots der so erhaltenen Teilbibliothek analog der vorgehensweiße von Ausführungsbeispiel 1 (Punkt 1.6) wurde die Menge von Plasmid-DNA bestimmt, welche notwendig ist, um nun ca. 5.000 transformierte. Klone mittels Elektroporation zu erhalten.

Anschließend wurde die bestimmte Menge der Teilbibliothek in den Expressionsstamm transformiert und das gleiche Verfahren wie für die ursprüngliche Bibliothek durchlaufen.

Da in dem ursprünglichen Well 1563 unterschiedliche Klone vorhanden waren und ca. 5000 Klone aufgeteilt wurden, sollte der die Adenosin-spaltende Aktivität zeigende Klone ca. 3-mal zu finden sein.

**Fig. 3** zeigt die erhaltenen Daten für diese Teilbibliothek. Es wurde ein Well mit einer sehr hohen Aktivität und drei weitere mit ebenfalls noch deutlich vom Hintergrund unterscheidbarer Aktivität detektiert, so dass der Klon 4-mal auf der Platte vorhanden war. Das Well mit dem höchsten Aktivitätswert wurde für den weiteren Vereinzelungsschritt ausgewählt. In diesem Well waren nun mehr nur noch 5000 / 96 = 52 unterschiedliche Klone vorhanden. Die Plasmide wurden wiederum in diesem Wells aus dem Bakterienpellet reisoliert.

5.2 Weitere Vereinzelungen - 2. Schritt

Eine weitere Wiederholung des dargestellten Schemas mit einer Aufteilung von jetzt ca. 500 Klonen ergab eine nochmals angereicherte Teilbibliothek mit durchschnittlich 250 / 96 = 5,2 Klonen pro Well. Der die Aktivität verursachende Klon konnte auf dieser Platte 10-mal wieder gefunden werden **(Fig. 4).** Von einem der Aktivität zeigendem Wells wurden wiederum die Plasmide aus dem Bakterienpellet isoliert.

5.3 Weitere Vereinzelungen - 3. Schritt

Ein Aliqout dieser Plasmidmischung wurde in den Expressionsstamm elektroporiert und die Transformanten auf einer Festagar-Platte ausgestrichen und die Platte über Nacht bei 37 °C inkubiert. Von den gewachsen Einzelklonen wurden 20 ausgewählt und damit direkt 100 µl Vorkulturen in einer MTP wie in 3.5. angesetzt. Nach Durchlaufen der Schritte 3.6.-3.10. konnte die detektierte Aktivität nun einem Einzelklon zugeordnet werden und der Genotyp der Adenosin-spaltenden RNaseT1-Variante identifiziert werden.

**Abkürzungsverzeichnis:**

In der Erfmdungsbeschreibung werden folgende Abkürzungen verwendet:

| | |
|---|---|
| *B. subtilis* | *Bacillus subtilis* |
| *C. lucknowese* | *Chrysosporium lucknowese* |
| Cy5 | Fluoreszenzfarbstoff Cy5™ (Amersham Biosciences UK Limited, Little Chalfont, Buckinghamshire, GB) |
| DEPC | Diethylpyrocarbonat |
| DWP | Deepwellplatte |
| *E. coli* | *Eschericha coli* |
| EDTA | Ethylendiamintetraessigsäure |
| h | Stunde |
| IPTG | Isopropyl-β-D-thiogalacto-pyranosid |
| LB | Luria Broth |
| MES | Morpholinoethansulfonsäure |
| min | Minuten |
| MTP | Microtiterplatte |
| OD | optische Dichte |
| $OD_{600}$ | optische Dichte bei 600 nm |
| ompA | äußeres Membranprotein A aus *E. coli* |
| p | Plasmid |
| PCR | Polymerase-Kettenreaktion |
| PT7 | T7-Promotor |
| rA | Riboadenylsäurerest |
| rG | Riboguanylsäurerest |
| Upm | Umdrehungen pro Minute |
| RhG | Rhodamin Grün (Fluoreszenzfarbstoff) |
| SAP | Alkalische Phosphatase aus Krabben |
| *S. cerevisiae* | *Saccharomyces cerevisiae* (Hefe) |
| Tris | Tris-(hydroxymethyl)-aminomethan |
| T4 | vom Bakteriophage T4 abstammend |
| U | Unit (Einheit für Enzymaktivität) |
| w/v | bei Prozentangaben: Gewicht (w = weight) pro Volumen (v) |

**Patentansprüche**

**1.** Verfahren zur Selektion von Biomolekülen aus Varianten-Bibliotheken von Biomolekülen umfassend die Schritte:

b) Aufteilung einer Vananten-Bibliothek, bestehend aus einer Anzahl ($B_0$) von Varianten in einem Größenbereich von $B_0 = 10^3$ bis $B_0 = 10^{15}$ der für das Biomolekül codierenden Gensequenz in eine Anzahl von $10^1$ bis $10^4$ Kompartimenten ($W_0$), die mindestens um einen Faktor 10 kleiner ist als die Anzahl ($B_0$) der in der Varianten-Bibliothek enthaltenen Varianten, wobei jedes Kompartiment eine Teilbibliothek enthält, die $K_0 \approx B_0/W_0$ Varianten enthält;
c) Vervielfältigung der Teilbibliothek in den Kompartimenten bis zu einer Individuen-Anzahl $V_0(x)$ zum Zeitpunkt x pro Kompartiment, wobei die Individuen-Anzahl $V_0(x)$ dividiert durch die Klonanzahl pro Kompartiment $K_0$ den Vervielfältigungsfaktor $F_0(x)$ pro Klon ergibt:

Produktion von Biomolekülen in den Kompartimenten vor, während oder nach der Vervielfältigung der Genotypen;
Konservierung eines Anteils der Teilbibliothek auf Organismen-Ebene oder der Ebene reiner codierender Sequenzen zum Zeitpunkt x unter Beibehaltung der Kompartiment Zuordnung: und
Test der in den einzelnen Kompartimenten erhaltenen Biomoleküle auf eine bestimmte Eigenschaft (Phänotyp), wobei Genotyp und Phänotyp entkoppelt sind und aus dem beobachteten Phänotyp keine direkten Rückschlüsse auf den Genotyp möglich sind:

d) Auswahl mindestens eines Kompartiments, in dem Biomoleküle enthalten sind, welche die gewünschten Eigenschaften erfüllen;
e) Verdünnung der entsprechend konservierten Teilbibliothek anhand des Faktors $F_0(x)$, so dass in einem gegebenen Volumen jedes in dem Kompartiment enthaltene Klon statistisch bis zu einer Anzahl $X_0 < W_1$ vorkommt; und Aufteilung dieses Volumens ohne vorherige Vervielfältigung auf neue Kompartimente $W_1$, wobei die neue Klonanzahl pro Kompartiment $K_1 = X_0 \neq K_0/W_1$ beträgt; und
f) n-faches WIederholen der Schritte c) bis e), bis in jedem Kompartiment nur noch maximal eine Variante ($K_n \leq 1$) der für das Biomolekül codierenden Gensequenz enthalten ist.

**2.** Verfahren nach Anspruch 1, **dadurch gekennzeichnet, dass** es den Schritt umfasst:

a) Herstellung der Varianten-Bibliothek.

**3.** Verfahren nach Anspruch 1 oder 2, **dadurch gekennzeichnet, dass** die gewünschte Eigenschaft eine biokatalytische Aktivität ist.

**4.** Verfahren nach einem der Ansprüche 1 bis 3, **dadurch gekennzeichnet, dass** die Variantenbibliothek vor der Aufteilung im Schritt b) in einen Organismus überführt wird.

5. Verfahren nach Anspruch 4, **dadurch gekennzeichnet, dass** die Kultur des Organismus nach der Aufteilung im Schritt c) auf eine Organismenzahl von $10^8$ bis $10^9$ pro Kompartiment vervielfältigt wird.

6. Verfahren nach Anspruch 4 oder 5, **dadurch gekennzeichnet, dass** die Organismen auch die Produktion der Biomoleküle durchführen.

7. Verfahren nach einem der Ansprüche 4 bis 6, **dadurch gekennzeichnet, dass** die Teilbibliotheken in den Kompartimenten aus den Organismen reisoliert werden und die Produktion der Biomoleküle durch zellfreie Systeme durchgeführt wird.

8. Verfahren nach einem der Ansprüche 1 bis 3, **dadurch gekennzeichnet, dass** die Vervielfältigung der Teilbibliotheken und die Produktion der Biomoleküle durch zellfreie Systeme durchgeführt wird.

9. Verfahren nach einem der Ansprüche 1 bis 8, **dadurch gekennzeichnet, dass** die Variantenbibliothek aus DNA-Plasmiden besteht, welche die für das Biomolekül codierende Gensequenz enthalten.

10. Verfahren nach einem der Ansprüche 1 bis 8, **dadurch gekennzeichnet, dass** die Variantenbibliothek aus linearen Nukleinsäuremolekülen besteht, welche die für das Biomolekül codierende Gensequenz enthalten.

11. Verfahren nach einem der Ansprüche 1 bis 10, **dadurch gekennzeichnet, dass** die Biomolekülen Enzyme oder Ribozyme oder andere Biomoleküle sind, welche eine biokatalytische Aktivität besitzen.

12. Verfahren nach einem der Ansprüche 1 bis 11, **dadurch gekennzeichnet, dass** der Test auf eine biokatalytische Aktivität mittels physikalischer Messmethoden, wie vorzugsweise der UV/VIS-Spektroskopie, der Fluoreszenz-Spektroskopie oder der Fluoreszenz-Korrelations-Spektroskopie, erfolgt.

13. Verfahren nach einem der Ansprüche 1 bis 12, **dadurch gekennzeichnet, dass** in der Ausgangsbibliothek mehr als 90% der erstellten Varianten nicht überlebensfähig sind.

14. Verfahren nach Anspruch 13, **dadurch gekennzeichnet, dass** in der Ausgangsbibliothek mehr als 99,9% der erstellten Varianten nicht überlebensfähig sind.

## Claims

1. Process for the selection of biomolecules from variant libraries of biomolecules comprising the steps of:

b) Division of a variant library consisting of a number of variants ($B_0$) of gene sequences coding for the biomolecule in the dimension of $B_0 = 10^3$ to $10^{15}$ into a number of $10^1$ to $10^4$ compartments ($W_0$), which is at least by a factor of ten smaller than the number of variants in the variant library ($B_0$), whereby each compartment contains a partial library which contains $K_0 = B_0/W_0$ variants;
c) Amplification of the partial library in the compartments up to a number of individuals $V_0(x)$ at the point in time x per compartment, whereas the number of individuals $V_0(x)$ divided by the number of clones per compartment $K_0$ gives the amplification factor $F_0(x)$ per clone;
Production of biomolecules in the compartments before, during or after amplification of the genotypes;
Conservation of a part of the partial library on the level of organisms or on the level of the pure coding sequences at the point in time x under retention of the compartment allocation;
Testing of the biomolecules obtained in the single compartments for a specified property (phenotype), whereas phenotype and genotype are decoupled and no direct conclusions on the genotype can be made from the observed phenotype,
d) Selection of at least one compartment, which contains biomolecules fulfilling the desired properties,
e) Dilution of the corresponding conserved partial library by means of the factor $F_0(x)$ so that in a given volume every clone contained in the compartment is statistically present up to a number $X_0 < W_1$, and division of this volume into a number of new compartments $W_1$ without prior amplification, whereby the new number of clones per compartment amounts to $K_1 = X_0 * K_0/W_1$; and
f) n-fold repetition of the steps c) to e) until in every compartment maximally only one variant ($K_1 \leq 1$) of the gene sequence coding for the biomolecule is contained.

2. The process according to claim 1, **characterized in that** the process comprises the step:

   a) Production of the variant library.

3. The process according to claim 1 or 2, **characterized in that** the desired property is a biocatalytic activity.

4. The process according to any one of the claims 1 to 3, **characterized in that** in step b) the variant library is transferred into an organism before division.

5. The process according to claim 4, **characterized in that** in step c) the culture of the organism is amplified to a number of organisms of $10^8$ to $10^9$ per compartment after division.

6. The process according to claim 4 or 5, **characterized in that** the organisms also conduct the production of the biomolecules.

7. The process according to any one of claims 4 to 6, **characterized in that** the partial libraries in the compartments are re-isolated from the organisms, and the production of the biomolecules is conducted by cell-free systems.

8. The process according to any one of claims 1 to 3, **characterized in that** the amplification of the partial libraries and the production of the biomolecules is conducted by cell-free systems.

9. The process according to any one of claims 1 to 8, **characterized in that** the variant library consists of DNA-plasmids, which contain the gene sequence coding for the biomolecule.

10. The process according to any one of claims 1 to 8, **characterized in that** the variant library consists of linear nucleic acid molecules, which contain the gene sequence coding for the biomolecule.

11. The process according to any one of claims 1 to 0, **characterized in that** the biomolecules are enzymes or ribozymes or other biomolecules, which exhibit a biocatalytic activity.

12. The process according to any one of claims 1 to 11, **characterized in that** the test for a biocatalytic activity is conducted with a physical detection method such as preferably UV/VIS-spectroscopy, fluorescence-spectroscopy or fluorescence-correlation-spectroscopy.

13. The process according to any one of claims 1 to 2, **characterized in that** within the starting library more than 90 % of the generated variants are not survivable.

14. The process according to claim 13, **characterized in that** within the starting library more than 99.9 % of the generated variants are not survivable.

## Revendications

1. Procédé pour la sélection de biomolécules dans des banques de variants de biomolécules, comprenant les étapes :

   b) subdivision d'une banque de variants, constituée d'un nombre ($B_0$) de variants dans une plage de $B_0 = 10^3$ à $B_0 = 10^{15}$ de la séquence génique codant pour la biomolécule, en un nombre de $10^1$ à $10^4$ compartiments ($W_0$), qui est d'au moins un facteur 10 plus petit que le nombre ($B_0$) des variants contenus dans la banque de variants, chaque compartiment contenant une banque partielle qui contient $K_0 \approx B_0/W_0$ variants ;
   c) amplification de la banque partielle dans les compartiments jusqu'à un nombre d'individus $V_0(x)$ à l'instant x par compartiment, le nombre d'individus $V_0(x)$, divisé par le nombre de clones par compartiment $K_0$, donnant le facteur d'amplification $F_0(x)$ par clone ;
   production de biomolécules dans les compartiments avant, pendant ou après l'amplification des génotypes ;
   conservation d'une partie de la banque partielle au niveau des organismes ou au niveau des séquences codantes pures, à l'instant x, avec maintien de l'affectation des compartiments ; et
   essai des biomolécules obtenues dans les compartiments individuels, portant sur une certaine propriété (phénotype), le génotype et le phénotype étant découplés, et aucune conclusion directe portant sur le phénotype n'étant possible à partir du phénotype observé ;

d) sélection d'au moins un compartiment dans lequel sont contenues des biomolécules qui satisfont aux propriétés souhaitées ;
e) dilution de la banque partielle conservée correspondante, à l'aide du facteur $F_0(x)$, de telle sorte que, dans un volume donné, chaque clone contenu dans le compartiment soit statistiquement présent jusqu'à un nombre $X_0 < W_1$, et subdivision de ce volume, sans amplification préalable, en de nouveaux compartiments $W_1$, le nouveau nombre de clones par compartiment étant $K_1 = X_0 \pm K_0/W_1$ ; et
f) répétition n fois des étapes c) à e) jusqu'à ce que chaque compartiment ne contienne encore au maximum qu'un variant ($K_n \leq 1$) de la séquence génique codant pour la biomolécule.

**2.** Procédé selon la revendication 1, **caractérisé en ce qu'**il comprend l'étape :

a) fabrication de la banque de variants.

**3.** Procédé selon la revendication 1 ou 2, **caractérisé en ce que** la propriété souhaitée est une activité biocatalytique.

**4.** Procédé selon l'une des revendications 1 à 3, **caractérisé en ce que** la banque de variants est transférée dans un organisme avant la subdivision de l'étape b).

**5.** Procédé selon la revendication 4, **caractérisé en ce que** la culture de l'organisme est, après la subdivision de l'étape c), amplifiée jusqu'à un nombre d'organismes de $10^8$ à $10^9$ par compartiment.

**6.** Procédé selon la revendication 4 ou 5, **caractérisé en ce que** les organismes réalisent aussi la production des biomolécules.

**7.** Procédé selon l'une des revendications 4 à 6, **caractérisé en ce que** les banques partielles sont, dans les compartiments, ré-isolés des organismes, et la production des biomolécules est réalisée par des systèmes sans cellules.

**8.** Procédé selon l'une des revendications 1 à 3, **caractérisé en ce que** l'amplification des banques partielles et la production des biomolécules sont réalisées par des systèmes sans cellules.

**9.** Procédé selon l'une des revendications 1 à 8, **caractérisé en ce que** la banque de variants est constituée d'ADN plasmidiques, qui contiennent la séquence génique codant pour la biomolécule.

**10.** Procédé selon l'une des revendications 1 à 8, **caractérisé en ce que** la banque de variants est constituée de molécules d'acides nucléiques linéaires, qui contiennent la séquence génique codant pour la biomolécule.

**11.** Procédé selon l'une des revendications 1 à 10, **caractérisé en ce que** les biomolécules sont des enzymes ou des ribozymes ou d'autres biomolécules qui possèdent une activité biocatalytique.

**12.** Procédé selon l'une des revendications 1 à 11, **caractérisé en ce que** l'essai portant sur une activité biocatalytique a lieu à l'aide de méthodes de mesure physiques telles que de préférence la spectroscopie UV/VIS, la spectroscopie en fluorescence ou la spectroscopique de corrélation de fluorescence.

**13.** Procédé selon l'une des revendications 1 à 12, **caractérisé en ce que**, dans la banque de départ, plus de 90 % des variants obtenus sont non viables.

**14.** Procédé selon la revendication 13, **caractérisé en ce que**, dans la banque de départ, plus de 99,9 % des variants obtenus sont non viables.

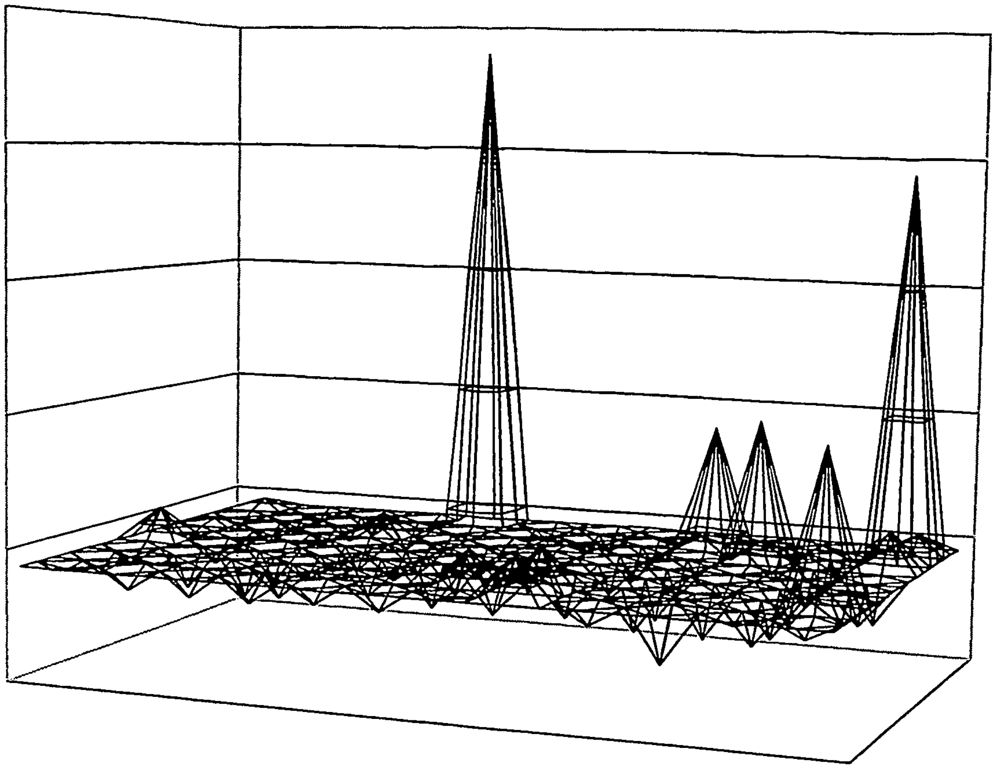

Fig. 1

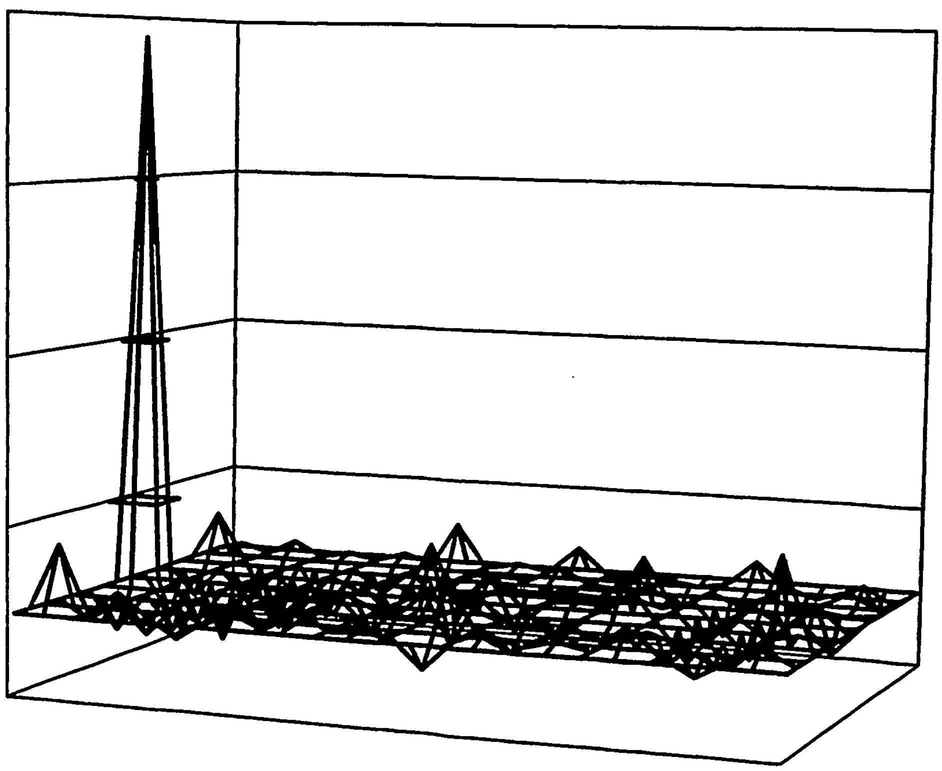

Fig. 2

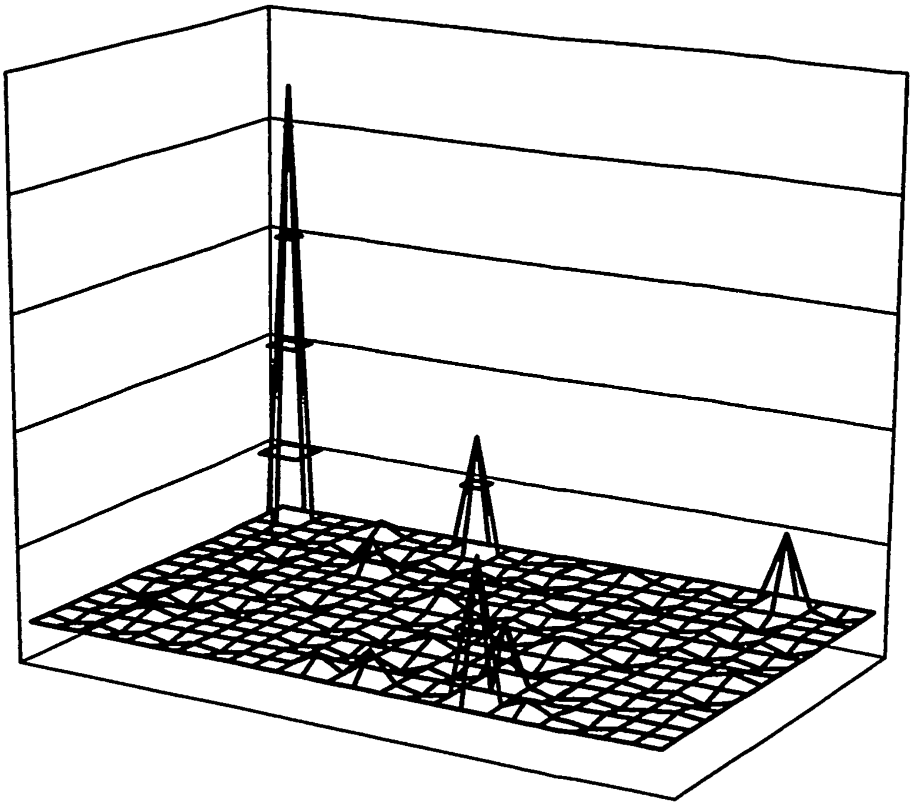

Fig. 3

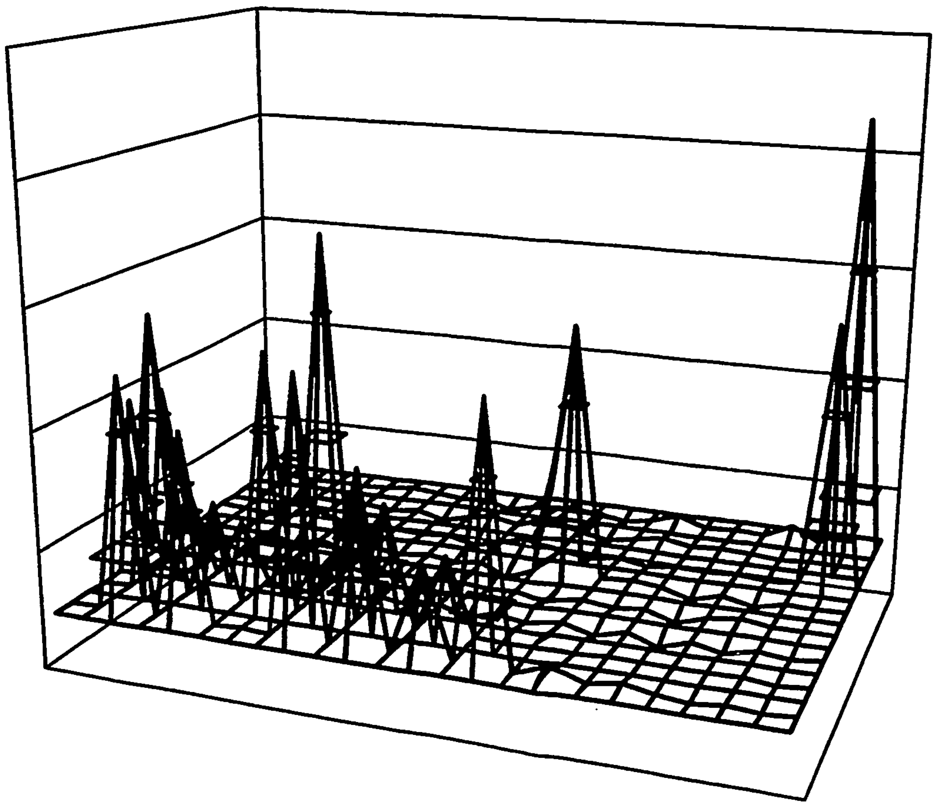

Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0112791 A
- DE 0019646372 C1
- DE 10350474


### In der Beschreibung aufgeführte Nicht-Patentliteratur


- **HUBNER, B. et al.** Modification of Ribonuclease T1 specificity by random mutagenesis of the substrate binding segment. *Biochemistry,* 1999, vol. 38, 1371-6
- **MARC, S.** *Veränderung der Substratspezifität von Ribonuklease T1 und der Einsatz des Enzyms in Immuntoxinen,* 2003
- **KORN, K. et al.** Analysis of the Rnase T1 mediated cleavage of an immobilized gapped heteroduplex via fluorescence correlation spectroscopy. *Biological Chemistry,* vol. 381, 259-63
- **KORN, K. et al.** Ribonuclease assays utilizing toluidine blue indicator plates, methylene blue, or fluorescence correlation spectroscopy. *Methods in Enzymology,* 2001, vol. 341, 142-53
- **EIGEN, M ; MCCASKILL, J. ; SCHUSTER, P.** The molecular quasispecies. *Adv. Chem. Phys,* 1989, vol. 75, 149-263
- The effect of high-frequency random mutagenesis on in vitro protein evolution: a study on TEM-1 beta-lactamase. *J. Mol. Biol.,* 1999, vol. 285, 775-83
- **SAMUELSON JC ; XU SY.** Directed evolution of restriction endonuclease Butyl to achieve increased substrate specificity. *J. Mol. Biol.,* 2002, vol. 319, 673-83
- **AMSTUTZ P ; FORRER P ; ZAHND C ; PLUCKTHUN A.** In vitro display technologies: novel developments and applications. *Curr. Opin.Biotechnol.,* 2001, vol. 12, 400-5
- **XIA G ; CHEN L ; SERA T ; FA M ; SCHULTZ PG ; ROMESBERG FE.** Directed evolution of novel polymerase activities: mutation of a DNA polymerase into an efficient RNA polymerase. *Proc. Natl. Acad. Sci. USA.,* 2002, vol. 99, 6597-602
- **JOO H ; LIN Z ; ARNOLD FH.** Laboratory evolution of peroxide-mediated cytochrome P450 hydroxylation. *Nature,* 1999, vol. 399, 670-3
- **KORBEL GA ; LALIC G ; SHAIR MD.** Reaction microarrays: a method for rapidly determining the enantiomeric excess of thousands of samples. *J. Am. Chem. Soc.,* 2001, vol. 123, 361-2
- **CADWELL RC ; JOYCE GF.** Randomization of genes by PCR mutagenesis. *PCR Methods Appl.,* 1992, vol. 2, 28-33
- **WELLS JA ; VASSER M ; POWERS DB.** Cassette mutagenesis: an efficient method for generation of multiple mutations at defined sites. *Gene,* 1985, vol. 34, 315-23